# EUROPÄISCHE PATENTSCHRIFT

(11) **EP 2 038 407 B1**
(45) Veröffentlichungstag und Bekanntmachung des Hinweises auf die Patenterteilung: **08.01.2014**
(21) Anmeldenummer: 07786846.1
(22) Anmeldetag: 26.06.2007
(51) Int. Cl.: C12P 7/62, C12P 41/00, C12N 9/16

(54) **PROTEINE MIT ESTERASE-AKTIVITÄT**
PROTEINS WITH ESTERASE ACTIVITY
PROTÉINE À ACTIVITÉ ESTÉRASE

(30) Priorität: 27.06.2006 EP 06013236
(43) Veröffentlichungstag der Anmeldung: 25.03.2009
(73) Patentinhaber: BASF SE, 67056 Ludwigshafen (DE)
(72) Erfinder: BREUER, Michael, 64285 Darmstadt (DE); HAUER, Bernhard, 67136 Fussgönheim (DE); SCHWANEBERG, Ulrich, 27721 Ritterhude (DE); WONG, Tuck, Seng, 28759 Bremen (DE)
(86) Internationale Anmeldenummer: PCT/EP2007/056367
(87) Internationale Veröffentlichungsnummer: WO 2008/000738

(56) Entgegenhaltungen:
- WO-A-02/18560
- DATABASE UniProt [Online] 24. Januar 2006 (2006-01-24), "Hydrolase, alpha/beta fold family." XP002453733 gefunden im EBI accession no. UNIPROT:Q2T897 Database accession no. Q2T897
- QUYEN DINH THI ET AL: "A novel esterase from Ralstonia sp. M1: gene cloning, sequencing, high-level expression and characterization." PROTEIN EXPRESSION AND PURIFICATION FEB 2007, Bd. 51, Nr. 2, 23. Juni 2006 (2006-06-23), Seiten 133-140, XP002453731 Epub ahead of print ISSN: 1046-5928

### SEQUENCE LISTING

<110> BASF Aktiengesellschaft
<120> Butinol I Esterase Mutants
<130> M/47154
<160> 21
<170> PatentIn version 3.3
<210> 1
   <211> 1530
   <212> DNA
   <213> Pseudomonas glumae
<220>
   <221> CDS
   <222> (1)..(1530)
<400> 1
   atg atc gtc caa ctg atc gcc atc gtg gtc gcc ctc tac gcc gtg ctg      48
   Met Ile Val Gln Leu Ile Ala Ile Val Val Ala Leu Tyr Ala Val Leu
   1               5                   10                  15
   ttc gcg ttc acg ctg ttc acc gcg cat cag gtg cgc cgc cgc ttt ccg      96
   Phe Ala Phe Thr Leu Phe Thr Ala His Gln Val Arg Arg Arg Phe Pro
               20                  25                  30
   ccc gag ggc aag ttc gtc gag atc gac ggc gac cgc ctg cat tat gtc      144
   Pro Glu Gly Lys Phe Val Glu Ile Asp Gly Asp Arg Leu His Tyr Val
           35                  40                  45
   gac tac ggc agc ggg ccg ccg atc gtg atg gtg cat ggc ctg tgc ggg      192
   Asp Tyr Gly Ser Gly Pro Pro Ile Val Met Val His Gly Leu Cys Gly
       50                  55                  60
   cag ctg ctg aac ttc gcc tac ctc gat ctg gcg cgg ctc gcg cag tcg      240
   Gln Leu Leu Asn Phe Ala Tyr Leu Asp Leu Ala Arg Leu Ala Gln Ser
   65                  70                  75                  80
   cat cgc gtg atc ctc gtc gat cgg gcc ggc tcg gga cgc tcg acg cgc      288
   His Arg Val Ile Leu Val Asp Arg Ala Gly Ser Gly Arg Ser Thr Arg
                   85                  90                  95
   ggc ccc gcc tcg cgc gcg aac gtc tat gcg cag gcg cgc ggc atc gcc      336
   Gly Pro Ala Ser Arg Ala Asn Val Tyr Ala Gln Ala Arg Gly Ile Ala
               100                 105                 110
   cgc ttc atc gag acg ctc ggc ctg gag cgg ccg gtg ctg gtg ggc cat      384
   Arg Phe Ile Glu Thr Leu Gly Leu Glu Arg Pro Val Leu Val Gly His
           115                 120                 125
   tcg ctc ggc ggc gcg atc gcg ctc gcg gtc ggc ctg gac tac ccc gag      432
   Ser Leu Gly Gly Ala Ile Ala Leu Ala Val Gly Leu Asp Tyr Pro Glu
       130                 135                 140
   cgc gtg agc cgc atc gcg ctg atc gcg ccg ctc acg cac acc gag acc      480
   Arg Val Ser Arg Ile Ala Leu Ile Ala Pro Leu Thr His Thr Glu Thr
   145                 150                 155                 160
   gag ccg ccc aag gcg ttc cgc ggg ctc gcg ctg cgc ccg gcg gcg ctg      528
   Glu Pro Pro Lys Ala Phe Arg Gly Leu Ala Leu Arg Pro Ala Ala Leu
                   165                 170                 175
   cgc cgc ttc gcg tcg ctg acg atg ggc atc ccg atc atg att ctg caa      576
   Arg Arg Phe Ala Ser Leu Thr Met Gly Ile Pro Ile Met Ile Leu Gln
               180                 185                 190
   agc cgc aag gcg atc gac gcg atc ttc gcg ccg gag ccg gtg ccg cgc      624
   Ser Arg Lys Ala Ile Asp Ala Ile Phe Ala Pro Glu Pro Val Pro Arg
           195                 200                 205
   gat ttc ccg ctg aag ggc ggc ggc atg atg ggg ctg cgg ccc gag gcg      672
   Asp Phe Pro Leu Lys Gly Gly Gly Met Met Gly Leu Arg Pro Glu Ala
       210                 215                 220
   ttc tac gcg gcg tcg tcg gac ctg gtc gcc gcg ccc gag gac ctg ccc      720
   Phe Tyr Ala Ala Ser Ser Asp Leu Val Ala Ala Pro Glu Asp Leu Pro
   225                 230                 235                 240
   gac atg gag cgc cgc tac ccg acg ctg ggc gtg ccg gtc agc atg ctg      768
   Asp Met Glu Arg Arg Tyr Pro Thr Leu Gly Val Pro Val Ser Met Leu
                   245                 250                 255
   tac ggg cgc cag gac gcg atc ctc gat ttc cac aag cat ggc gag ggg      816
   Tyr Gly Arg Gln Asp Ala Ile Leu Asp Phe His Lys His Gly Glu Gly
               260                 265                 270
   ctc aag cgc aag ctc gac ggc gtc gag ctg agc gcc gtc gag ggc ggg      864
   Leu Lys Arg Lys Leu Asp Gly Val Glu Leu Ser Ala Val Glu Gly Gly
           275                 280                 285
   cac atg ctg ccc gtg acg cag ccg gcc gcc acc acc gac tgg ctc ctc      912
   His Met Leu Pro Val Thr Gln Pro Ala Ala Thr Thr Asp Trp Leu Leu
       290                 295                 300
   gcg gtg gcc gcg gcg gcg aac gcg gcg gcg cag cac gat gcg gcg cgg      960
   Ala Val Ala Ala Ala Ala Asn Ala Ala Ala Gln His Asp Ala Ala Arg
   305                 310                 315                 320
   ccg gat ccg gca ccg tcc gag gtc acg cag gcc ggc gcg ctg cag cat     1008
   Pro Asp Pro Ala Pro Ser Glu Val Thr Gln Ala Gly Ala Leu Gln His
                   325                 330                 335
   ctg aag gtc ggc gac aac gtg ctg atc ggc aag aag ccc acc ggc acg     1056
   Leu Lys Val Gly Asp Asn Val Leu Ile Gly Lys Lys Pro Thr Gly Thr
               340                 345                 350
   ctg gtg gcc gac aac ctg ctg ccg ggc aag acc ctg tgg ctg ctg tcg     1104
   Leu Val Ala Asp Asn Leu Leu Pro Gly Lys Thr Leu Trp Leu Leu Ser
           355                 360                 365
   acc ggc acg ggt ctc gcg ccg ttc atg tcg atc atc cgc gat ccg gac     1152
   Thr Gly Thr Gly Leu Ala Pro Phe Met Ser Ile Ile Arg Asp Pro Asp
       370                 375                 380
   atc tac gaa cgc tac gag aag gtg gtg ctc acg cac acc tgc cgc ctg     1200
   Ile Tyr Glu Arg Tyr Glu Lys Val Val Leu Thr His Thr Cys Arg Leu
   385                 390                 395                 400
   aag ggc gag ctc gcg tac atg gac ttc atc aag cac gac ctg ccg ggc      1248
   Lys Gly Glu Leu Ala Tyr Met Asp Phe Ile Lys His Asp Leu Pro Gly
                   405                 410                 415
   cat gag tac ctc ggc gac atc atc aag gaa aag ctg atc tac tac ccg      1296
   His Glu Tyr Leu Gly Asp Ile Ile Lys Glu Lys Leu Ile Tyr Tyr Pro
               420                 425                 430
   acc gtc acg cgc gaa gcg ttc gac aac gag ggg cgg atc acc gac ctg      1344
   Thr Val Thr Arg Glu Ala Phe Asp Asn Glu Gly Arg Ile Thr Asp Leu
           435                 440                 445
   atc tcg acg ggc aag ctg ttc acc gat ctc gac gtc ccg ccg ttc tcg      1392
   Ile Ser Thr Gly Lys Leu Phe Thr Asp Leu Asp Val Pro Pro Phe Ser
       450                 455                 460
   ccc gag aac gac cgc gtg atg ctg tgc ggc agc acc gcg atg ctg aag      1440
   Pro Glu Asn Asp Arg Val Met Leu Cys Gly Ser Thr Ala Met Leu Lys
   465                 470                 475                 480
   gac acc acc gac ctg ctc aag cag gcc ggc ctc gtc gaa ggc aag aac      1488
   Asp Thr Thr Asp Leu Leu Lys Gln Ala Gly Leu Val Glu Gly Lys Asn
                   485                 490                 495
   agc gcg ccg ggc cac tat gtg atc gaa cgc gca ttt gtc gac              1530
   Ser Ala Pro Gly His Tyr Val Ile Glu Arg Ala Phe Val Asp
               500                 505                 510
<210> 2
   <211> 510
   <212> PRT
   <213> Pseudomonas glumae
<400> 2
   Met Ile Val Gln Leu Ile Ala Ile Val Val Ala Leu Tyr Ala Val Leu
   1               5                   10                  15
   Phe Ala Phe Thr Leu Phe Thr Ala His Gln Val Arg Arg Arg Phe Pro
               20                  25                  30
   Pro Glu Gly Lys Phe Val Glu Ile Asp Gly Asp Arg Leu His Tyr Val
           35                  40                  45
   Asp Tyr Gly Ser Gly Pro Pro Ile Val Met Val His Gly Leu Cys Gly
       50                  55                  60
   Gln Leu Leu Asn Phe Ala Tyr Leu Asp Leu Ala Arg Leu Ala Gln Ser
   65                  70                  75                  80
   His Arg Val Ile Leu Val Asp Arg Ala Gly Ser Gly Arg Ser Thr Arg
                   85                  90                  95
   Gly Pro Ala Ser Arg Ala Asn Val Tyr Ala Gln Ala Arg Gly Ile Ala
               100                 105                 110
   Arg Phe Ile Glu Thr Leu Gly Leu Glu Arg Pro Val Leu Val Gly His
           115                 120                 125
   Ser Leu Gly Gly Ala Ile Ala Leu Ala Val Gly Leu Asp Tyr Pro Glu
       130                 135                 140
   Arg Val Ser Arg Ile Ala Leu Ile Ala Pro Leu Thr His Thr Glu Thr
   145                 150                 155                 160
   Glu Pro Pro Lys Ala Phe Arg Gly Leu Ala Leu Arg Pro Ala Ala Leu
                   165                 170                 175
   Arg Arg Phe Ala Ser Leu Thr Met Gly Ile Pro Ile Met Ile Leu Gln
               180                 185                 190
   Ser Arg Lys Ala Ile Asp Ala Ile Phe Ala Pro Glu Pro Val Pro Arg
           195                 200                 205
   Asp Phe Pro Leu Lys Gly Gly Gly Met Met Gly Leu Arg Pro Glu Ala
       210                 215                 220
   Phe Tyr Ala Ala Ser Ser Asp Leu Val Ala Ala Pro Glu Asp Leu Pro
   225                 230                 235                 240
   Asp Met Glu Arg Arg Tyr Pro Thr Leu Gly Val Pro Val Ser Met Leu
                   245                 250                 255
   Tyr Gly Arg Gln Asp Ala Ile Leu Asp Phe His Lys His Gly Glu Gly
               260                 265                 270
   Leu Lys Arg Lys Leu Asp Gly Val Glu Leu Ser Ala Val Glu Gly Gly
           275                 280                 285
   His Met Leu Pro Val Thr Gln Pro Ala Ala Thr Thr Asp Trp Leu Leu
       290                 295                 300
   Ala Val Ala Ala Ala Ala Asn Ala Ala Ala Gln His Asp Ala Ala Arg
   305                 310                 315                 320
   Pro Asp Pro Ala Pro Ser Glu Val Thr Gln Ala Gly Ala Leu Gln His
                   325                 330                 335
   Leu Lys Val Gly Asp Asn Val Leu Ile Gly Lys Lys Pro Thr Gly Thr
                   340                 345                 350
   Leu Val Ala Asp Asn Leu Leu Pro Gly Lys Thr Leu Trp Leu Leu Ser
           355                 360                 365
   Thr Gly Thr Gly Leu Ala Pro Phe Met Ser Ile Ile Arg Asp Pro Asp
       370                 375                 380
   Ile Tyr Glu Arg Tyr Glu Lys Val Val Leu Thr His Thr Cys Arg Leu
   385                 390                 395                 400
   Lys Gly Glu Leu Ala Tyr Met Asp Phe Ile Lys His Asp Leu Pro Gly
                   405                 410                 415
   His Glu Tyr Leu Gly Asp Ile Ile Lys Glu Lys Leu Ile Tyr Tyr Pro
               420                 425                 430
   Thr Val Thr Arg Glu Ala Phe Asp Asn Glu Gly Arg Ile Thr Asp Leu
           435                 440                 445
   Ile Ser Thr Gly Lys Leu Phe Thr Asp Leu Asp Val Pro Pro Phe Ser
       450                 455                 460
   Pro Glu Asn Asp Arg Val Met Leu Cys Gly Ser Thr Ala Met Leu Lys
   465                 470                 475                 480
   Asp Thr Thr Asp Leu Leu Lys Gln Ala Gly Leu Val Glu Gly Lys Asn
                   485                 490                 495
   Ser Ala Pro Gly His Tyr Val Ile Glu Arg Ala Phe Val Asp
               500                 505                 510
<210> 3
   <211> 32
   <212> PRT
   <213> Artificial
<220>
   <223> Partial Sequence
<400> 3
   Phe Ile Glu Thr Leu Gly Leu Glu Arg Pro Val Leu Val Gly His Ser
   1               5                   10                  15
   Leu Gly Gly Ala Ile Ala Leu Ala Val Gly Leu Asp Tyr Pro Glu Arg
               20                  25                  30
<210> 4
   <211> 14
   <212> PRT
   <213> Artificial
<220>
   <223> Partial Sequence
<400> 4
   Ile Ala Leu Ile Ala Pro Leu Thr His Thr Glu Thr Glu Pro
   1               5                   10
<210> 5
   <211> 20
   <212> PRT
   <213> Artificial
<220>
   <223> Partial Sequeence
<400> 5
   Gly Gly Gly Met Met Gly Leu Arg Pro Glu Ala Phe Tyr Ala Ala Ser
   1               5                   10                  15
   Ser Asp Leu Val
               20
<210> 6
   <211> 11
   <212> PRT
   <213> Artificial
<220>
   <223> Partial Sequence
<400> 6
   Ala Ile Asp Ala Ile Phe Ala Pro Glu Pro Val
   1               5                   10
<210> 7
   <211> 1005
   <212> DNA
   <213> Pseudomonas glumae
<220>
   <221> CDS
   <222> (1)..(1005)
<400> 7
   atg atc gtc caa ctg atc gcc atc gtg gtc gcc ctc tac gcc gtg ctg        48
   Met Ile Val Gln Leu Ile Ala Ile Val Val Ala Leu Tyr Ala Val Leu
   1               5                   10                  15
   ttc gcg ttc acg ctg ttc acc gcg cat cag gtg cgc cgc cgc ttt ccg      96
   Phe Ala Phe Thr Leu Phe Thr Ala His Gln Val Arg Arg Arg Phe Pro
               20                  25                  30
   ccc gag ggc aag ttc gtc gag atc gac ggc gac cgc ctg cat tat gtc      144
   Pro Glu Gly Lys Phe Val Glu Ile Asp Gly Asp Arg Leu His Tyr Val
           35                  40                  45
   gac tac ggc agc ggg ccg ccg atc gtg atg gtg cat ggc ctg tgc ggg      192
   Asp Tyr Gly Ser Gly Pro Pro Ile Val Met Val His Gly Leu Cys Gly
       50                  55                  60
   cag ctg ctg aac ttc gcc tac ctc gat ctg gcg cgg ctc gcg cag tcg      240
   Gln Leu Leu Asn Phe Ala Tyr Leu Asp Leu Ala Arg Leu Ala Gln Ser
   65                  70                  75                  80
   cat cgc gtg atc ctc gtc gat cgg gcc ggc tcg gga cgc tcg acg cgc      288
   His Arg Val Ile Leu Val Asp Arg Ala Gly Ser Gly Arg Ser Thr Arg
                   85                  90                  95
   ggc ccc gcc tcg cgc gcg aac gtc tat gcg cag gcg cgc ggc atc gcc      336
   Gly Pro Ala Ser Arg Ala Asn Val Tyr Ala Gln Ala Arg Gly Ile Ala
               100                 105                 110
   cgc ttc atc gag acg ctc ggc ctg gag cgg ccg gtg ctg gtg ggc cat      384
   Arg Phe Ile Glu Thr Leu Gly Leu Glu Arg Pro Val Leu Val Gly His
           115                 120                 125
   tcg ctc ggc ggc gcg atc gcg ctc gcg gtc ggc ctg gac tac ccc gag      432
   Ser Leu Gly Gly Ala Ile Ala Leu Ala Val Gly Leu Asp Tyr Pro Glu
       130                 135                 140
   cgc gtg agc cgc atc gcg ctg atc gcg ccg ctc acg cac acc gag acc      480
   Arg Val Ser Arg Ile Ala Leu Ile Ala Pro Leu Thr His Thr Glu Thr
   145                 150                 155                 160
   gag ccg ccc aag gcg ttc cgc ggg ctc gcg ctg cgc ccg gcg gcg ctg      528
   Glu Pro Pro Lys Ala Phe Arg Gly Leu Ala Leu Arg Pro Ala Ala Leu
                   165                 170                 175
   cgc cgc ttc gcg tcg ctg acg atg ggc atc ccg atc atg att ctg caa      576
   Arg Arg Phe Ala Ser Leu Thr Met Gly Ile Pro Ile Met Ile Leu Gln
               180                 185                 190
   agc cgc aag gcg atc gac gcg atc ttc gcg ccg gag ccg gtg ccg cgc      624
   Ser Arg Lys Ala Ile Asp Ala Ile Phe Ala Pro Glu Pro Val Pro Arg
           195                 200                 205
   gat ttc ccg ctg aag ggc ggc ggc atg atg ggg ctg cgg ccc gag gcg      672
   Asp Phe Pro Leu Lys Gly Gly Gly Met Met Gly Leu Arg Pro Glu Ala
       210                 215                 220
   ttc tac gcg gcg tcg tcg gac ctg gtc gcc gcg ccc gag gac ctg ccc      720
   Phe Tyr Ala Ala Ser Ser Asp Leu Val Ala Ala Pro Glu Asp Leu Pro
   225                 230                 235                 240
   gac atg gag cgc cgc tac ccg acg ctg ggc gtg ccg gtc agc atg ctg      768
   Asp Met Glu Arg Arg Tyr Pro Thr Leu Gly Val Pro Val Ser Met Leu
                   245                 250                 255
   tac ggg cgc cag gac gcg atc ctc gat ttc cac aag cat ggc gag ggg      816
   Tyr Gly Arg Gln Asp Ala Ile Leu Asp Phe His Lys His Gly Glu Gly
                   260                 265                 270
   ctc aag cgc aag ctc gac ggc gtc gag ctg agc gcc gtc gag ggc ggg      864
   Leu Lys Arg Lys Leu Asp Gly Val Glu Leu Ser Ala Val Glu Gly Gly
           275                 280                 285
   cac atg ctg ccc gtg acg cag ccg gcc gcc acc acc gac tgg ctc ctc      912
   His Met Leu Pro Val Thr Gln Pro Ala Ala Thr Thr Asp Trp Leu Leu
       290                 295                 300
   gcg gtg gcc gcg gcg gcg aac gcg gcg gcg cag cac gat gcg gcg cgg      960
   Ala Val Ala Ala Ala Ala Asn Ala Ala Ala Gln His Asp Ala Ala Arg
   305                 310                 315                 320
   ccg gat ccg gca ccg tcc gag gtc acg cag gcc ggc gcg ctg cag         1005
   Pro Asp Pro Ala Pro Ser Glu Val Thr Gln Ala Gly Ala Leu Gln
                   325                 330                 335
<210> 8
   <211> 335
   <212> PRT
   <213> Pseudomonas glumae
<400> 8
   Met Ile Val Gln Leu Ile Ala Ile Val Val Ala Leu Tyr Ala Val Leu
   1               5                   10                  15
   Phe Ala Phe Thr Leu Phe Thr Ala His Gln Val Arg Arg Arg Phe Pro
               20                  25                  30
   Pro Glu Gly Lys Phe Val Glu Ile Asp Gly Asp Arg Leu His Tyr Val
           35                  40                  45
   Asp Tyr Gly Ser Gly Pro Pro Ile Val Met Val His Gly Leu Cys Gly
       50                  55                  60
   Gln Leu Leu Asn Phe Ala Tyr Leu Asp Leu Ala Arg Leu Ala Gln Ser
   65                  70                  75                  80
   His Arg Val Ile Leu Val Asp Arg Ala Gly Ser Gly Arg Ser Thr Arg
                   85                  90                  95
   Gly Pro Ala Ser Arg Ala Asn Val Tyr Ala Gln Ala Arg Gly Ile Ala
               100                 105                 110
   Arg Phe Ile Glu Thr Leu Gly Leu Glu Arg Pro Val Leu Val Gly His
           115                 120                 125
   Ser Leu Gly Gly Ala Ile Ala Leu Ala Val Gly Leu Asp Tyr Pro Glu
       130                 135                 140
   Arg Val Ser Arg Ile Ala Leu Ile Ala Pro Leu Thr His Thr Glu Thr
   145                 150                 155                 160
   Glu Pro Pro Lys Ala Phe Arg Gly Leu Ala Leu Arg Pro Ala Ala Leu
                   165                 170                 175
   Arg Arg Phe Ala Ser Leu Thr Met Gly Ile Pro Ile Met Ile Leu Gln
               180                 185                 190
   Ser Arg Lys Ala Ile Asp Ala Ile Phe Ala Pro Glu Pro Val Pro Arg
           195                 200                 205
   Asp Phe Pro Leu Lys Gly Gly Gly Met Met Gly Leu Arg Pro Glu Ala
       210                 215                 220
   Phe Tyr Ala Ala Ser Ser Asp Leu Val Ala Ala Pro Glu Asp Leu Pro
   225                 230                 235                 240
   Asp Met Glu Arg Arg Tyr Pro Thr Leu Gly Val Pro Val Ser Met Leu
                   245                 250                 255
   Tyr Gly Arg Gln Asp Ala Ile Leu Asp Phe His Lys His Gly Glu Gly
               260                 265                 270
   Leu Lys Arg Lys Leu Asp Gly Val Glu Leu Ser Ala Val Glu Gly Gly
           275                 280                 285
   His Met Leu Pro Val Thr Gln Pro Ala Ala Thr Thr Asp Trp Leu Leu
       290                 295                 300
   Ala Val Ala Ala Ala Ala Asn Ala Ala Ala Gln His Asp Ala Ala Arg
   305                 310                 315                 320
   Pro Asp Pro Ala Pro Ser Glu Val Thr Gln Ala Gly Ala Leu Gln
                   325                 330                 335
<210> 9
   <211> 24
   <212> DNA
   <213> Artificial
<220>
   <223> PCR Primer
<400> 9
   tttttgccct cgttatctag attt                                              24
<210> 10
   <211> 33
   <212> DNA
   <213> Artificial
<220>
   <223> PCR Primer
<400> 10
   ccggaattcc ggtatctaac taagcttgac ctg 33
<210> 11
   <211> 23
   <212> DNA
   <213> Artificial
<220>
   <223> PCR Primer
<400> 11
   gaccatgatt acgccaagct tgc 23
<210> 12
   <211> 33
   <212> DNA
   <213> Artificial
<220>
   <223> PCR Primer
<400> 12
   ccggaattcc ggtcactgca gcgcgccggc ctg 33
<210> 13
   <211> 27
   <212> DNA
   <213> Artificial
<220>
   <223> PCR Primer
<400> 13
   cgacaaaaat ctagataacg agggcaa 27
<210> 14
   <211> 27
   <212> DNA
   <213> Artificial
<220>
   <223> PCR Primer
<400> 14
   ttcacttcac aggtcaagct tagttag 27
<210> 15
   <211> 33
   <212> DNA
   <213> Artificial
<220>
   <223> PCR Primer
<220>
   <221> misc_feature
   <222> (16)..(18)
   <223> n is a, c, g, or t
<400> 15
   ggcatcccga tcatgnnnct gcaaagccgc aag 33
<210> 16
   <211> 33
   <212> DNA
   <213> Artificial
<220>
   <223> PCR Primer
<220>
   <221> misc_feature
   <222> (16)..(18)
   <223> n is a, c, g, or t
<400> 16
   cttgcggctt tgcagnnnca tgatcgggat gcc 33
<210> 17
   <211> 33
   <212> DNA
   <213> Artificial
<220>
   <223> PCR Primer
<220>
   <221> misc_feature
   <222> (16)..(18)
   <223> n is a, c, g, or t
<400> 17
   gggcgccagg acgcgnnnct cgatttccac aag 33
<210> 18
   <211> 33
   <212> DNA
   <213> Artificial
<220>
   <223> PCR Primer
<220>
   <221> misc_feature
   <222> (16)..(18)
   <223> n is a, c, g, or t
<400> 18
   cttgtggaaa tcgagnnncg cgtcctggcg ccc 33
<210> 19
   <211> 3246
   <212> DNA
   <213> Artificial
<220>
   <223> pASK-IBA7
<400> 19
   ccatcgaatg gccagatgat taattcctaa tttttgttga cactctatca ttgatagagt      60
   tattttacca ctccctatca gtgatagaga aaagtgaaat gaatagttcg acaaaaatct     120
   agataacgag ggcaaaaaat ggctagctgg agccacccgc agttcgaaaa aatcgaaggg     180
   cgccgagacc gcggtcccga attcgagctc ggtacccggg gatccctcga ggtcgacctg     240
   cagggggacc atggtctctg atatctaact aagcttgacc tgtgaagtga aaaatggcgc    300
   acattgtgcg acattttttt tgtctgccgt ttaccgctac tgcgtcacgg atctccacgc     360
   gccctgtagc ggcgcattaa gcgcggcggg tgtggtggtt acgcgcagcg tgaccgctac     420
   acttgccagc gccctagcgc ccgctccttt cgctttcttc ccttcctttc tcgccacgtt     480
   cgccggcttt ccccgtcaag ctctaaatcg ggggctccct ttagggttcc gatttagtgc     540
   tttacggcac ctcgacccca aaaaacttga ttagggtgat ggttcacgta gtgggccatc     600
   gccctgatag acggtttttc gccctttgac gttggagtcc acgttcttta atagtggact     660
   cttgttccaa actggaacaa cactcaaccc tatctcggtc tattcttttg atttataagg     720
   gattttgccg atttcggcct attggttaaa aaatgagctg atttaacaaa aatttaacgc     780
   gaattttaac aaaatattaa cgcttacaat ttcaggtggc acttttcggg gaaatgtgcg     840
   cggaacccct atttgtttat ttttctaaat acattcaaat atgtatccgc tcatgagaca     900
   ataaccctga taaatgcttc aataatattg aaaaaggaag agtatgagta ttcaacattt     960
   ccgtgtcgcc cttattccct tttttgcggc attttgcctt cctgtttttg ctcacccaga    1020
   aacgctggtg aaagtaaaag atgctgaaga tcagttgggt gcacgagtgg gttacatcga    1080
   actggatctc aacagcggta agatccttga gagttttcgc cccgaagaac gttttccaat    1140
   gatgagcact tttaaagttc tgctatgtgg cgcggtatta tcccgtattg acgccgggca    1200
   agagcaactc ggtcgccgca tacactattc tcagaatgac ttggttgagt actcaccagt    1260
   cacagaaaag catcttacgg atggcatgac agtaagagaa ttatgcagtg ctgccataac 1320
   catgagtgat aacactgcgg ccaacttact tctgacaacg atcggaggac cgaaggagct 1380
   aaccgctttt ttgcacaaca tgggggatca tgtaactcgc cttgatcgtt gggaaccgga 1440
   gctgaatgaa gccataccaa acgacgagcg tgacaccacg atgcctgtag caatggcaac 1500
   aacgttgcgc aaactattaa ctggcgaact acttactcta gcttcccggc aacaattgat 1560
   agactggatg gaggcggata aagttgcagg accacttctg cgctcggccc ttccggctgg 1620
   ctggtttatt gctgataaat ctggagccgg tgagcgtggc tctcgcggta tcattgcagc 1680
   actggggcca gatggtaagc cctcccgtat cgtagttatc tacacgacgg ggagtcaggc 1740
   aactatggat gaacgaaata gacagatcgc tgagataggt gcctcactga ttaagcattg 1800
   gtaggaatta atgatgtctc gtttagataa aagtaaagtg attaacagcg cattagagct 1860
   gcttaatgag gtcggaatcg aaggtttaac aacccgtaaa ctcgcccaga agctaggtgt 1920
   agagcagcct acattgtatt ggcatgtaaa aaataagcgg gctttgctcg acgccttagc 1980
   cattgagatg ttagataggc accatactca cttttgccct ttagaagggg aaagctggca 2040
   agatttttta cgtaataacg ctaaaagttt tagatgtgct ttactaagtc atcgcgatgg 2100
   agcaaaagta catttaggta cacggcctac agaaaaacag tatgaaactc tcgaaaatca 2160
   attagccttt ttatgccaac aaggtttttc actagagaat gcattatatg cactcagcgc 2220
   agtggggcat tttactttag gttgcgtatt ggaagatcaa gagcatcaag tcgctaaaga 2280
   agaaagggaa acacctacta ctgatagtat gccgccatta ttacgacaag ctatcgaatt 2340
   atttgatcac caaggtgcag agccagcctt cttattcggc cttgaattga tcatatgcgg 2400
   attagaaaaa caacttaaat gtgaaagtgg gtcttaaaag cagcataacc tttttccgtg 2460
   atggtaactt cactagttta aaaggatcta ggtgaagatc ctttttgata atctcatgac 2520
   caaaatccct taacgtgagt tttcgttcca ctgagcgtca gaccccgtag aaaagatcaa 2580
   aggatcttct tgagatcctt tttttctgcg cgtaatctgc tgcttgcaaa caaaaaaacc 2640
   accgctacca gcggtggttt gtttgccgga tcaagagcta ccaactcttt ttccgaaggt 2700
   aactggcttc agcagagcgc agataccaaa tactgtcctt ctagtgtagc cgtagttagg 2760
   ccaccacttc aagaactctg tagcaccgcc tacatacctc gctctgctaa tcctgttacc 2820
   agtggctgct gccagtggcg ataagtcgtg tcttaccggg ttggactcaa gacgatagtt 2880
   accggataag gcgcagcggt cgggctgaac ggggggttcg tgcacacagc ccagcttgga 2940
   gcgaacgacc tacaccgaac tgagatacct acagcgtgag ctatgagaaa gcgccacgct 3000
   tcccgaaggg agaaaggcgg acaggtatcc ggtaagcggc agggtcggaa caggagagcg 3060
   cacgagggag cttccagggg gaaacgcctg gtatctttat agtcctgtcg ggtttcgcca   3120
   cctctgactt gagcgtcgat ttttgtgatg ctcgtcaggg gggcggagcc tatggaaaaa   3180
   cgccagcaac gcggcctttt tacggttcct ggccttttgc tggccttttg ctcacatgac   3240
   ccgaca                                                              3246
<210> 20
   <211> 30
   <212> DNA
   <213> Artificial
<220>
   <223> PCR Primer
<400> 20
   ggcgagaagc ttagaaatca tgatcgtcca 30
<210> 21
   <211> 30
   <212> DNA
   <213> Artificial
<220>
   <223> PCR Primer
<400> 21
   ggatcctcta gagtctcact gcagcgcgcc 30

## Beschreibung

Die Erfindung betrifft neuartige Proteine mit Esterase-Aktivität, funktionale Äquivalente und Mutanten davon, dafür kodierende Nukleinsäuresequenzen, Expressionskassetten, Vektoren und rekombinante Mikroorganismen; Verfahren zur Herstellung dieser Proteine und deren Verwendung zur enzymatischen, insbesondere enantioselektiven enzymatischen Esterhydrolyse oder Umesterung organischer Ester.

### Hintergrund der Erfindung

Esterasen und Lipasen gehören zu den Hydrolasen, die zur Synthese optisch aktiver, organischer Verbindungen in technischen Prozessen eingesetzt werden können und sich durch hohe Substratspezifität auszeichnen. Sie können in einem den Serinproteasen ähnlichen Mechanismus Acylgruppen auf Nukleophile, wie z. B. Carbonylgruppen, übertragen oder Esterbindungen hydrolytisch spalten. Den Esterasen, Lipasen und Serinproteasen ist die katalytische Triade gemeinsam, ein Sequenzmotiv aus den Aminosäuren Ser, His und Asp, wobei das aktive Ser das Carbonylkohlenstoffatom nukleophil angreift und es unter Beteiligung der anderen beiden Aminosäuren zu einer Ladungsverteilung kommt. Esterasen und Lipasen können Acylgruppen auch auf andere Nukleophile, wie Thiogruppen eines Thioethers oder aktivierte Amine, übertragen.

Lipasen hydrolysieren langkettige Glycerinester und zeichnen sich durch Grenzflächenaktivierung aus, d. h. dass das aktive Zentrum nur in Gegenwart des Lipidsubstrats zugänglich wird. Lipasen sind in nicht-wässrigen organischen Lösungsmitteln stabil und werden in zahlreichen technischen Prozessen zur kinetischen Racematspaltung eingesetzt, d. h. ein Enantiomer wird wesentlich schneller als das andere umgesetzt. Dieses lässt sich nachfolgend aufgrund unterschiedlicher physikalischer und chemischer Eigenschaften aus der Reaktionslösung gewinnen.

Nakamura (Nakamura, K. et al., Tetrahedron; Asymmetry 9, (1999), 4429-4439) beschreibt die Racematspaltung von 1-Alkyn-3-ol durch Umesterung mit Hilfe käuflich erhältlicher Lipasen (Amano AK, AH und PS; Amano Pharmaceuticals Co. Ltd.) in hydrophoben Lösungsmitteln, wobei die Enantioselektivität mit der Kettenlänge des Acyldonors steigt und sterisch große Reste (Chloracetat, Vinylbenzoat) die Reaktion negativ beeinflussen. Yang (Yang, H. et al., J. Org. Chem. 64, (1999), 1709-1712) beschreibt die enantioselektive Herstellung optisch aktiver Säuren durch Umesterung mit Vinylestern unter Verwendung der Lipase B aus Candida antarctica als Katalysator. Ethylester führen hier zu einer deutlich geringeren Reaktionsgeschwindigkeit und Selektivität. Eine aus Burkholderia plantarii (Pseudomonas plantarii oder glumae) DSM 6535 isolierte Lipase wird zur enantioselektiven Acylierung racemischer Amine mit Hilfe von Ethylmethoxyacetat eingesetzt (Balkenhohl, F. et al., J. prakt. Chem. 339, (1997), 381-384).

Esterasen katalysieren enantioselektiv die Bildung und Auflösung von Esterbindungen (Hin- und Rückreaktion). Bei der Umesterung zur Gewinnung optisch aktiver Alkohole werden vorzugsweise Vinylester eingesetzt, da die Alkoholfunktion des Esters nach der Umsetzung durch Tautomerisierung zu Aldehyd oder Keton nicht mehr zur Verfügung steht und damit die Rückreaktion vermieden werden kann. Esterasen sind im Gegensatz zu Lipasen nicht grenzflächenaktiviert und setzen auch organische Verbindungen kürzerer Kettenlänge um. Aus verschiedenen Organismen wurden Esterasen unterschiedlicher Substratspezifität isoliert.

So findet die Esterase aus Pseudocardia thermophila FERM-BP-6275 Verwendung bei der Hydrolyse optisch aktiver Chromanessigsäureester (EP-A-0 892 044).

Eine Esterase aus Bacillus acidocaldarius hydrolysiert Ester eines engen Substratspektrums und mit niedriger Enantioselektivität (Manco, G. et al., Biochem. J. 332, (1998), 203-212).

Acylase 1 aus Aspergillus wird zur Gewinnung sekundärer Alkohole durch Umesterung mit Vinylestern in organischen unpolaren Lösungsmitteln verwendet, wobei bevorzugt sekundäre Alkohole mit kurzen Seitenketten umgesetzt werden (Faraldos, J. et al., Synlett 4, (1997), 367-370). Aus Pseudomonas fluorescens DSM 50 106 wurde eine Membran-gebundene Lacton-spezifische Esterase kloniert (Khalameyzer, V. et al., Appl. and Environ. Microbiol. 65(2), (1999), 477-482), und aus der Q-Mutante von E. coli eine Acetylesterase (Peist, R. et al., J. Bacteriol. 179, (1997), 7679-7686). Jedoch wurden Enantioselektivität und Substratspezifität dieser beiden Esterasen nicht näher untersucht. Rhodococcus sp. NCBM 11216 exprimiert 4 Esterasen, RR1 bis RR4, die unterschiedliche Spezifität aufweisen. Bei der Estersynthese aus Naphthol. und einer Säure bevorzugen RR1 und RR2 Säuren mit kurzen Kohlenstoffketten, während RR3 und RR4 spezifisch Säuren mit längeren Kohlenstoffketten und sterisch größeren Resten umsetzen (Gudelj, M. et al., J. Mol. Cat. B, Enzymatic 5, (1998), 261-266).

Zur Herstellung kleiner organischer Moleküle, wie optisch aktiver Alkohole, Säuren oder Ester mit kurzen Kohlenstoffketten, standen bis vor kurzem jedoch keine Esterasen zur Verfügung, die ein breites Substratspektrum besitzen, eine hohe Enantioselektivität aufweisen und in technischen Prozessen eingesetzt werden können.

In der WO-A-02/18560 wurden erstmalig brauchbare Proteine mit Esteraseaktivität, dort als Esterasen bezeichnet, beschrieben, die zur enantioselektiven Hydrolyse eines breiten Spektrums optisch aktiver Ester befähigt sind. Insbesondere wurde dort ein 510 Aminosäuren unfassendes Protein (SEQ ID NO:2) und dessen kodierende Sequenz (SEQ ID NO: 1) beschrieben.

Aufgabe der vorliegenden Erfindung ist die Bereitstellung von weiteren gegebenenfalls aktivitätsoptimierten Esterasen, welche wenigstens eine der oben genannten Eigenschaften aufweisen.

### Kurzfassung der Erfindung

Diese Aufgabe wurde überraschenderweise durch die Bereitstellung eines Proteins mit Esterase-Aktivität, oder Mutanten davon gemäß der Definition in den Patentansprüchen gelöst.

### Figurenbeschreibung

Figur 1 zeigt ein Sequenz-Alignment einer Aminosäure-Teilsequenz der Butinol I Esterase mit einer Teilsequenz einer Lacton-spezifischen Esterase aus Pseudomonas fluorescens. Query: Teil-Sequenz des erfindungsgemäßen Klones LU2898. Sbjct: Teil-Sequenz des P. fluorescens Enzyms, (Accession No.: O87637)
Figur 2 veranschaulicht das Klonierungsschema für die LU2898.
Figur 3 zeigt den Vergleich des Butinolesterasegens aus LU2898 mit der ERGO-Datenbank. Bereiche mit hoher Homologie sind dunkel dargestellt. In den rechten Textspalten sind die Annotationen aus ERGO und die entsprechenden e-Werte angeben; der e-Wert gibt die Wahrscheinlichkeit an, mit der die Sequenzhomologie Zufallsbasiert ist.. Die Lage der *Pst*I Schnittstelle im Butinolesterasegen ist markiert. Im Vergleich dazu die Aminosäuresequenz des nicht-rekombinanten Enzyms aus *Ps. glumae* LU2023.
Figur 4 veranschaulicht die enzymatische Aktivität einer erfindungsgemäßen Esterase mit einer Sequenzlänge von 335 Aminosäuren (aus Klon LU11147) und davon abgeleiteter aktivitätsverbesserter Mutanten.

### Detaillierte Beschreibung der Erfindung

### 1. Bevorzugte Ausführungsformen

Ein erster Gegenstand der Erfindung betrifft ein Protein mit Esterase-Aktivität oder ein funktional äquivalentes Protein davon, nach Anspruch 1, mit einem berechneten Molekulargewicht von etwa 38 bis 34 kDa oder 36,5 bis 35,5 kDa, wobei das Protein vorzugsweise eine Esterase-Aktivität besitzt, die durch Spaltung des Esters But-3-yn-2-yl-butyrat als Referenzsubstrat charakterisierbar ist.

Erfindungsgemäße Proteine können insbesondere zusätzlich wenigstens eine weitere Aminosäure-Teilsequenz gemäß SEQ ID NO: 3, 4, 5 oder 6 umfassen.

Die genannten Aminosäure-Teilsequenzen gemäß SEQ ID NO: 3, 4, 5 oder 6 sind wie folgt definiert (jeweils angegeben im Aminosäure-Einbuchstabencode, wobei die jeweils erste Aminosäure dem jeweiligen aminoterminalen Ende entspricht):
a) FIETLGLERPVLVGHSLGGAIALAVGLDYPER (SEQ ID NO:3),
b) IALIAPLTHTETEP (SEQ ID NO:4),
c) GGGMMGLRPEAFYAASSDLV (SEQ ID NO:5)
d) AIDAIFAPEPV (SEQ ID NO:6)

Die erfindungsgemäßen Proteine umfassen insbesondere eine Polypeptidkette mit einer Gesamtlänge von weniger als 350 bis 335 Aminosäuren, wie insbesondere 345 bis 335, vor allem 335 Aminosäuren.

Besonders genannt sei ein Protein, umfassend eine Aminosäuresequenz gemäß SEQ ID NO:8.

Ein weiterer Gegenstand der Erfindung betrifft aktivitätsverbesserte Mutanten dieser neuen verkürzten Esterasen, sowie analog hergestellte Mutanten der aus der WO-A-02/18560 bekannten, etwa 510 Aminosäuren umfassenden Esterasen und funktionalen Äquivalenten davon, wie in den Ansprüchen definiert.

Gegenstand der Erfindung sind insbesondere Esterase-Mutanten welche wenigstens eine funktionale Mutation in einem der Aminosäuresequenzbereiche 12-20, 185-195 und 258 bis 268 von SEQ ID NO:2 oder 8 und vor allem wenigstens eine funktionale Mutation in einer der Aminosäuresequenzpositionen 16, 190 und 263 von SEQ ID NO:2 oder 8 aufweisen.

Nicht- limitierende Beispiele solcher Mutationen sind folgende Aminosäuresubstitutionen: Leu16Pro, Ile190Thr, Ile190Arg und Ile 263Val, einzeln oder in beliebiger Kombination.

Die erfindungsgemäßen Mutanten sind außerdem dadurch gekennzeichnet, dass sie eine Polypeptidkette mit einem berechneten Molekulargewicht von etwa 60 kDa oder weniger, wie z.B. 56 kDa oder weniger oder 55,5 kDa oder weniger. Beispielsweise können die verkürzten Butinolesterasen mit weniger als 510 Aminosäuren ein Molekulargewicht im Bereich von etwa 56 kDa bis 20 kDa, wie z.B. etwa 55,5 bis 30 kDa oder 55,5 bis 35 kDa oder 55,5 bis 40 kDa oder 40 bis 30 kDa, oder 55,5 bis 45 kDa oder 38 bis 34 kDa oder 36,5 bis 35,5 kDa aufweisen.

Mutanten von Proteinen gemäß SEQ ID NO: 2 weisen bevorzugt berechnete Molekulargewichte im Bereich von etwa 60 bis 40 kDa, oder 56 bis 50 kDa oder 55,5 bis 54 kDa auf.

Mutanten von Proteinen gemäß SEQ ID NO: 8 weisen bevorzugt berechnete Molekulargewichte im Bereich von etwa 38 bis 34 kDa, 37 bis 35 kDa oder 36,5 bis 35,5 kDa auf.

Bevorzugte Proteine oder Mutanten sind aus Pseudomonas glumae (auch bezeichnet als Burkholderia plantarii) Lu 2023 mit der Hinterlegungsnummer DSM 13176 (hinterlegt bei der DSMZ am 2. Dezember 1999) und gegebenenfalls anschließende Mutation erhältlich.

Die erfindungsgemäßen Proteine mit Esterase-Aktivität, funktionalen Äquivalente und Mutanten sind außerdem dadurch gekennzeichnet, dass wenigstens eine der folgenden Reaktionen katalysieren:
a) enantioselektive Hydrolyse optisch aktiver Ester der Formel I

   R¹-COO-R² (I),

   worin
   R¹ für geradkettiges oder verzweigtes, gegebenenfalls ein- oder mehrfach substituiertes C₁-C₁₀-Alkyl, C₂-C₁₀-Alkenyl, C₂-C₁₀-Alkinyl und R² für geradkettiges oder verzweigtes, gegebenenfalls ein- oder mehrfach substituiertes C₁-C₁₀-Alkyl, C₂-C₁₀-Alkeny), C₂-C₁₀-Alkinyl, C₇-C₁₅-Aralkyl oder für einen ein- oder mehrkernigen, gegebenenfalls ein- oder mehrfach substituierten aromatischen Rest steht,
   R¹ und/oder R² wenigstens ein asymmetrisches Kohlenstoffatom umfasst; und
b) enantioselektive Umesterung eines Esters der Formel I mit einem optisch aktiven Alkohol der Formel II

   R²-OH (II),

   worin R² eine der oben stehenden Bedeutungen besitzt, und gegebenenfalls wenigstens ein asymmetrisches Kohlenstoffatom aufweist.

Als Beispiele für geeignete C₁-C₁₀-Alkylreste sind zu nennen geradkettige oder verzweigte Reste mit 1 bis 10 C-Atomen, wie Methyl, Ethyl, i- oder n-Propyl, n-, i-, sec.-oder tert.-Butyl, n- oder i-Pentyl; außerdem n-Hexyl, n-Heptyl, n-Octyl, n-Nonyl, n-Decyl, sowie die ein- oder mehrfach verzweigten Analoga davon.

Als Beispiele für geeignete C₂-C₁₀-Alkenylreste sind die ein- oder mehrfach ungesättigt, vorzugsweise eine oder zwei C-C-Doppelbindungen aufweisende Analoga oben genannter Alkylreste mit 2 bis 10 Kohlenstoffatomen zu nennen, wobei die Doppelbindung in beliebiger Position der Kohlenstoffkette liegen kann.

Als Beispiele für geeignete C₂-C₁₀-Alkinylreste sind die ein- oder mehrfach ungesättigt, vorzugsweise ein oder zwei C-C-Dreifachbindungen aufweisenden ungesättigten Analoga oben genannter Alkylreste mit 2 bis 10 Kohlenstoffatomen zu nennen, wobei die Dreifachbindung in beliebiger Position der Kohlenstoffkette liegen kann.

C₇-C₁₅-Aralkyl steht vorzugsweise für Phenyl-C₁-C₅-alkyl- oder Naphthyl-C₁-C₅-alkyl-.

Als Beispiele für einen ein- oder mehrkernigen, gegebenenfalls ein- oder mehrfach substituierten aromatischen Rest sind zu nennen Phenyl und Naphthyl welch mit 1, 2 oder 3 gleichen oder verschiedenen Substituenten ausgewählt unter C₁-C₅-Alkyl, wie Methyl, Ethyl, i- oder n-Propyl, n-, i-, sec.- oder tert.-Butyl, n- oder i-Pentyl; Hydroxy, Mercapto, Amino, Nitro oder Halogen, wie F, Br, Cl, substituiert sind.

Der Ester der Formel I ist beispielsweise abgeleitet von geradkettigen oder verzweigten, gegebenenfalls ein- oder mehrfach ungesättigten, gegebenenfalls substituierten C₁-C₁₁-Monocarbonsäuren. Beispielsweise sind zu nennen: Gesättigten Säuren, wie Ameisen-, Essig-, Propion- und n- und i-Buttersäure, n- und i-Valeriansäure, Capronsäure, Önanthsäure, Caprylsäure, Pelargonsäure, Caprinsäure, Undecansäure; einfach ungesättigte Säuren, wie Acrylsäure, Crotonsäure; und zweifach ungesättigten Säuren, wie Sorbinsäure. Sind in den Säuren Doppelbindungen enthalten, so können diese sowohl in cis- als auch in trans-Form vorliegen.

Gegenstand der Erfindung sind weiterhin Polynukleotide, die für ein Protein, ein funktionales Äquivalent oder eine Mutante gemäß obiger Definition kodieren, sowie funktionale Äquivalente dieser Polynukleotide, dazu komplementäre Polynukleotide, und die damit hybridisierbaren Nukleinsäuresequenzen.

Insbesondere sind Gegenstand solche Polynukleotide, umfassend eine Nukleotidsequenz gemäß SEQ ID NO:7.

Gegenstand der Erfindung ist außerdem ein Polynukleotid, wobei das Codon in dem Bereich entsprechend der Aminosäureposition 263 von SEQ ID NO:8 ausgewählt ist unter GTT und GTC.

Ein weiterer Gegenstand der Erfindung betrifft Expressionskassetten, umfassend wenigstens ein Polynukleotid gemäß obiger Definition, operativ verknüpft mit wenigstens einer regulatorischen Nukleinsäuresequenz.

Ein weiterer Gegenstand der Erfindung betrifft rekombinante Vektoren zur Transformation eines eukaryontischen oder prokaryontischen Wirts, umfassend ein Polynukleotid gemäß obiger Definition, oder eine Expressionskassette gemäß obiger Definition.

Ferner betrifft die Erfindung ein Verfahren zur Herstellung eines Proteins gemäß obiger Definition, wobei man einen Mikroorganismus, welcher das Protein endogen produziert, oder einen mit einem Vektor gemäß obiger Definition transformierten Mikroorganismus kultiviert und das Protein aus der Kultur isoliert.

Geeignete Verfahren verwenden dabei den Mikroorganismus Pseudomonas glumae (Burkholderia plantarii) Lu 2023 mit der Hinterlegungsnummer DSM 13176 oder einen davon abgeleiteten Mikroorganismus.

Gegenstand der Erfindung sind auch Protein gemäß obiger Definition, sowie deren funktionalen Äquivalente oder Mutanten, erhältlich nach einem Verfahren gemäß obiger Definition.

Weiterhin betrifft die Erfindung Pseudomonas glumae (Burkholderia plantarii) Lu 2023 mit der Hinterlegungsnummer DSM 13176 und Varianten und Mutanten davon.

Gegenstand der Erfindung sind außerdem Mikroorganismen, dadurch gekennzeichnet, dass er einen Vektor gemäß obiger Definition tragen.

Weiterhin betrifft die Erfindung ein Verfahren zur enantioselektiven Esterhydrolyse unter Verwendung eines Proteins (einschließlich funktionaler Äquivalente und Mutanten) gemäß obiger Definition, wobei
a) das Protein mit einem Stereoisomerengemisch eines optisch aktiven Esters der Formel I in Kontakt gebracht wird; und
b) die bei der stereoselektiven Hydrolyse eines der Stereoisomeren anfallenden optisch aktiven Verbindungen und/oder das nicht hydrolysierte Esterenantiomer aus dem Reaktionsmedium gewonnen wird.

Gegenstand der Erfindung ist auch ein Verfahren zur enantioselektiven Umesterung, wobei
a) ein Stereoisomerengemisch eines optisch aktiven Alkohols der Formel II mit einem Ester der Formel I in Gegenwart eines Proteins (einschließlich funktionaler Äquivalente und Mutanten) gemäß obiger Definition in Kontakt gebracht wird und das nicht umgesetzte Alkoholstereoisomer aus dem Reaktionsmedium gewonnen wird; oder
b) ein Stereoisomerengemisch eines optisch aktiven Esters der Formel I mit einem Alkohol der Formel II in Gegenwart eines Proteins (einschließlich funktionaler Äquivalente und Mutanten) gemäß obiger Definition in Kontakt gebracht wird und ein Stereoisomer des im Ester enthaltenen optisch aktiven Alkohols aus dem Reaktionsmedium gewonnen wird.

Gemäß einer besonderen Variante dieses Verfahrens verwendet man zur Umesterung einen Vinylester als Acylierungsmittel für einen optisch aktiven Alkohol.

Insbesondere ist Gegenstand ein Verfahren gemäß obiger Definition, wobei als Reaktionsmedium ein organisches Lösungsmittel verwendet wird.

### 2. Erläuterung allgemeiner Begriffe

Eine "Esterase" oder "Butinol Esterase" oder "Butinol I Esterase" ist ein Enzym welches wenigstens eine der hierin angegebenen enzymatischen Umsetzungen, insbesondere wenigstens die Spaltung eines Referenz-Butinolesters einer Carbonsäure, insbesondere der Buttersäure, wie z.B. eines Butinol-Butyrats, wie But-3yn-2yl-butyrat, katalysiert.

Unter einer von einer konkret offenbarten Sequenz "abgeleiteten" oder dazu "homologen" Sequenz, z.B. einer abgeleiteten Aminosäure- oder Nukleinsäuresequenz, wird erfindungsgemäß, wenn keine anderen Angaben gemacht werden, eine Sequenz verstanden, die mit der Ausgangssequenz eine Identität von mindestens 80% oder mindestens 90%, insbesondere 91%, 92%, 93%, 94%, 95%, 96%, 97%, 98% und 99% aufweist.

Unter "Identität" zwischen zwei Nukleinsäuren wird die Identität der Nukleotide über die jeweils gesamte Nukleinsäurelänge verstanden, insbesondere die Identität, die durch Vergleich mit Hilfe der Vector NTI Suite 7.1 Software der Firma Informax (USA) unter Anwendung der Clustal Methode (Higgins DG, Sharp PM. Fast and sensitive multiple sequence alignments on a microcomputer. Comput Appl. Biosci. 1989 Apr;5(2):151-1) unter Einstellung folgender Parameter berechnet wird:

### Multiple alignment parameter:

| | |
|---|---|
| Gap opening penalty | 10 |
| Gap extension penalty | 10 |
| Gap separation penalty range | 8 |
| Gap separation penalty | off |
| % identity for alignment delay | 40 |
| Residue specific gaps | off |
| Hydrophilic residue gap | off |
| Transition weighing | 0 |

### Pairwise alignment parameter:

| | |
|---|---|
| FAST algorithm on | |
| K-tuple size | 1 |
| Gap penalty | 3 |
| Window size | 5 |
| Number of best diagonals | 5 |

### 3. Weitere Ausgestaltungen der Erfindung

### 3.1 Erfindungsgemäße Proteine

Die vorliegende Erfindung ist nicht auf die konkret offenbarten Proteine bzw. Enzyme mit Esteraseaktivität beschränkt, sondern erstreckt sich vielmehr auch auf funktionale Äquivalente davon.

"Funktionale Äquivalente" oder Analoga der konkret offenbarten Enzyme sind im Rahmen der vorliegenden Erfindung davon verschiedene Polypeptide, welche weiterhin die gewünschte biologische Aktivität, wie z.B. hydrolytische Aktivität, besitzen.

So versteht man beispielsweise unter "funktionalen Äquivalenten" Enzyme, die im verwendeten Test auf Esterase-Aktivität eine um mindestens 1%, wie z.B. mindestens 10% oder 20 %, wie z.B. mindestens 50 % oder 75% oder 90 % höhere oder niedrigere Aktivität eines Enzyms, umfassend eine Aminosäuresequenz gemäß SEQ ID NO: 8 aufweisen. Funktionale Äquivalente sind außerdem vorzugsweise zwischen pH 4 bis 10 stabil und besitzen vorteilhaft ein pH-Optimum in Einem Bereich von pH 5 bis 9, wie z.B. 6 bis 8, sowie ein Temperaturoptimum im Bereich von 15°C bis 80°C. oder 20°C bis 70°C.

Die Esterase-Aktivität kann mit Hilfe verschiedener bekannter Tests nachgewiesen werden. Ohne darauf beschränkt zu sein, sei ein Test unter Verwendung eines Referenzsubstrates, wie z. B. eines Butinol-butyrats, wie But-3yn-2ylbutyrat, unter standardisierten Bedingungen (wie z. B. 20 mM Substrat, 10 mM Phosphatpuffer, pH 7,4 T = 20 °C) zu nennen.

Unter "funktionalen Äquivalenten" versteht man erfindungsgemäß insbesondere auch "Mutanten", welche in wenigstens einer Sequenzposition der oben genannten Aminosäuresequenzen eine andere als die konkret genannte Aminosäure aufweisen aber trotzdem eine der oben genannten biologischen Aktivitäten besitzen. "Funktionale Äquivalente" umfassen somit die durch eine oder mehrere Aminosäure-Additionen, - Substitutionen, -Deletionen und/oder -Inversionen erhältlichen Mutanten, wobei die genannten Veränderungen in jeglicher Sequenzposition auftreten können, solange sie zu einer Mutante mit dem erfindungsgemäßen Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche Substrate mit unterschiedlicher Geschwindigkeit umgesetzt werden. Beispiele für geeignete Aminosäuresubstitutionen sind in folgender Tabelle zusammengefasst:

| Ursprünglicher Rest | | Beispiele der Substitution | |
|---|---|---|---|
| | Ala | | Ser |
| | Arg | | Lys |
| | Asn | | Gln; His |
| | Asp | | Glu |
| | Cys | | Ser |
| | Gln | | Asn |
| | Glu | | Asp |
| | Gly | | Pro |
| | His | | Asn ; Gln |
| | Ile | | Leu; Val |
| | Leu | | Ile; Val |
| | Lys | | Arg ; Gln ; Glu |
| | Met | | Leu ; Ile |
| | Phe | | Met ; Leu ; Tyr |
| | Ser | | Thr |
| | Thr | | Ser |
| | Trp | | Tyr |
| | Tyr | | Trp ; Phe |
| | Val | | Ile; Leu |

Konkrete Beispiele einzelner zur Durchführung erfindungsgemäßer Mutationen geeigneter sequenzbereiche sind ober bereits definiert worden, und zwar: die Aminosäuresequenzbereiche 12-20, 185-195 und 258 bis 268 von SEQ ID NO:2 oder 8., insbesondere die Aminosäuresequenzpositionen 16, 190 und 263 von SEQ ID NO:2 oder 8.

Konkrete Beispiele geeigneter Aminosäuresubstitutionen sind Leu16Pro, Ile190Thr, Ile190Arg und Ile263Val. Weitere Abwandlungen davon können vom Fachmann in Kenntnis der Lehre der vorliegenden Erfindung ohne weiteres bereitgestellt werden.

"Funktionale Äquivalente" im obigen Sinne sind auch "Präkursoren" der beschriebenen Polypeptide sowie "funktionale Derivate" und "Salze" der Polypeptide.

"Präkursoren" sind dabei natürliche oder synthetische Vorstufen der Polypeptide mit oder ohne der gewünschten biologischen Aktiviät.

Unter dem Ausdruck "Salze" versteht man sowohl Salze von Carboxylgruppen als auch Säureadditionssalze von Aminogruppen der erfindungsgemäßen Proteinmoleküle. Salze von Carboxylgruppen können in an sich bekannter Weise hergestellt werden und umfassen anorganische Salze, wie zum Beispiel Natrium-, Calcium-, Ammonium-, Eisen- und Zinksalze, sowie Salze mit organischen Basen, wie zum Beispiel Aminen, wie Triethanolamin, Arginin, Lysin, Piperidin und dergleichen. Säureadditionssalze, wie zum Beispiel Salze mit Mineralsäuren, wie Salzsäure oder Schwefelsäure und Salze mit organischen Säuren, wie Essigsäure und Oxalsäure sind ebenfalls Gegenstand der Erfindung.

"Funktionale Derivate" erfindungsgemäßer Polypeptide können an funktionellen Aminosäure-Seitengruppen oder an deren N- oder C-terminalen Ende mit Hilfe bekannter Techniken ebenfalls hergestellt werden. Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylgruppen; oder O-Acylderivate freier Hydroxygruppen, hergestellt durch Umsetzung mit Acylgruppen.

"Funktionale Äquivalente" umfassen natürlich auch Polypeptide welche aus anderen Organismen zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.

"Funktionale Äquivalente" umfassen ebenfalls Fragmente, vorzugsweise einzelne Domänen oder Sequenzmotive, der erfindungsgemäßen Polypeptide, welche z.B. die gewünschte biologische Funktion aufweisen.

"Funktionale Äquivalente" sind außerdem Fusionsproteine, welche eine der oben genannten Polypeptidsequenzen oder davon abgeleitete funktionale Äquivalente und wenigstens eine weitere, davon funktionell verschiedene, heterologe Sequenz in funktioneller N- oder C-terminaler Verknüpfung (d.h. ohne gegenseitigen wesentliche funktionelle Beeinträchtigung der Fusionsproteinteile) aufweisen. Nichtlimitierende Beispiele für derartige heterologe Sequenzen sind z.B. Signalpeptide, Histidin-Anker oder Enzyme.

Erfindungsgemäß mit umfasste "funktionale Äquivalente" sind Homologe zu den konkret offenbarten Proteinen. Diese besitzen wenigstens 60 %, vorzugsweise wenigstens 75% ins besondere wenigsten 85 %, wie z.B. 90, 91, 92, 93, 94, 95, 96, 97,98 oder 99%, Homologie zu einer der konkret offenbarten Aminosäuresequenzen, berechnet nach dem Algorithmus von Pearson und Lipman, Proc. Natl. Acad, Sci. (USA) 85(8), 1988, 2444-2448. Eine prozentuale Homologie eines erfindungsgemäßen homologen Polypeptids bedeutet insbesondere prozentuale Identität der Aminosäurereste bezogen auf die Gesamtlänge einer der hierin konkret beschriebenen Aminosäuresequenzen.

Im Falle einer möglichen Proteinglykosylierung umfassen erfindungsgemäße "funktionale Äquivalente" Proteine des oben bezeichneten Typs in deglykosylierter bzw. glykosylierter Form sowie durch Veränderung des Glykosylierungsmusters erhältliche abgewandelte Formen.

Homologe der erfindungsgemäßen Proteine oder Polypeptide können durch Mutagenese erzeugt werden, z.B. durch Punktmutation, Verlängerung oder Verkürzung des Proteins.

Homologe der erfindungsgemäßen Proteine können durch Screening kombinatorischer Banken von Mutanten, wie z.B. Verkürzungsmutanten, identifiziert werden. Beispielsweise kann eine variegierte Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen kodieren. Verfahren zur Synthese degenerierter Oligonukleotide sind dem Fachmann bekannt (z.B. Narang, S.A. (1983) Tetrahedron 39:3; Itakura et al. (1984) Annu. Rev. Biochem. 53:323; Itakura et al., (1984) Science 198:1056; Ike et al. (1983) Nucleic Acids Res. 11:477).

Im Stand der Technik sind mehrere Techniken zum Screening von Genprodukten kombinatorischer Banken, die durch Punktmutationen oder Verkürzung hergestellt worden sind, und zum Screening von cDNA-Banken auf Genprodukte mit einer ausgewählten Eigenschaft bekannt. Diese Techniken lassen sich an das schnelle Screening der Genbanken anpassen, die durch kombinatorische Mutagenese erfindungsgemäßer Homologer erzeugt worden sind. Die am häufigsten verwendeten Techniken zum Screening großer Genbanken, die einer Analyse mit hohem Durchsatz unterliegen, umfassen das Klonieren der Genbank in replizierbare Expressionsvektoren, Transformieren der geeigneten Zellen mit der resultierenden Vektorenbank und Exprimieren der kombinatorischen Gene unter Bedingungen, unter denen der Nachweis der gewünschten Aktivität die Isolation des Vektors, der das Gen kodiert, dessen Produkt nachgewiesen wurde, erleichtert. Recursive-Ensemble-Mutagenese (REM), eine Technik, die die Häufigkeit funktioneller Mutanten in den Banken vergrößert, kann in Kombination mit den Screeningtests verwendet werden, um Homologe zu identifizieren (Arkin und Yourvan (1992) PNAS 89:7811-7815; Delgrave et al. (1993) Protein Engineering 6(3):327-331).

Weitere Beispiele funktionaler Äquivalente der erfindungsgemäßen Esterasen umfassen z. B. wenigstens eine von SEQ ID NO: 3, 4, 5 oder 6 abgeleitete Teilsequenz, wobei in Vergleich zur konkret angegebenen Teilsequenz eine oder mehrere Aminosäuren substituiert, deletiert, invertiert oder addiert sind, und wobei sich die Esteraseaktivität nicht um mehr als ±90 % oder ± 50 %, vorzugsweise nicht um mehr als ± 30 % von der Esteraseaktivität des nativen Proteins (SEQ ID NO:8) unterscheidet.

Die erfindungsgemäßen Esterasen sind insbesondere aus Pseudomonas glumae LU2023 der Hinterlegungsnummer DSM 13176 erhältlich. Weitere Stammvarianten sind z.B. ausgehend von Pseudomonas glumae LU8093 durch Selektion, wie beispielsweise durch Kultur auf Minimalmedium-Platten, mit Ethylphenylacetat als einziger Kohlenstoffquelle, zugänglich.

### 3.2 Kodierende Nukleinsäuresequenzen

Gegenstand der Erfindung sind auch Nukleinsäuresequenzen, die für ein Enzym mit Esterase-Aktivität kodieren. Bevorzugt sind Nukleinsäuresequenzen, umfassend eine Sequenz gemäß SEQ ID NO:7; oder eine von der Aminosäuresequenz gemäß SEQ ID NO.: 8 abgeleitete Nukleinsäuresequenz.

Alle hierin erwähnten Nukleinsäuresequenzen (einzel- und doppelsträngige DNA- und RNA-Sequenzen, wie z.B. cDNA und mRNA) sind in an sich bekannter Weise durch chemische Synthese aus den Nukleotidbausteinen, wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seiten 896-897) erfolgen. Die Anlagerung synthetischer Oligonukleotide und Auffüllen von Lücken mit Hilfe des Klenow-Fragmentes der DNA-Polymerase und Ligationsreaktionen sowie allgemeine Klonierungsverfahren werden in Sambrook et al. (1989), Molecular Cloning: A laboratory manual, Cold Spring Harbor Laboratory Press, beschrieben.

Gegenstand der Erfindung sind auch Nukleinsäuresequenzen (einzel- und doppelsträngige DNA- und RNA-Sequenzen, wie z.B. cDNA und mRNA), kodierend für eines der obigen Polypeptide und deren funktionalen Äquivalenten, welche z.B. unter Verwendung künstlicher Nukleotidanaloga zugänglich sind.

Die Erfindung betrifft sowohl isolierte Nukleinsäuremoleküle, welche für erfindungsgemäße Polypeptide bzw. Proteine oder biologisch aktive Abschnitte davon kodieren, als auch Nukleinsäurefragmente, die z.B. zur Verwendung als Hybridisierungssonden oder Primer zur Identifizierung oder Amplifizierung von erfindungsgemäßer kodierenden Nukleinsäuren verwendet werden können.

Die erfindungsgemäßen Nukleinsäuremoleküle können zudem untranslatierte Sequenzen vom 3'- und/oder 5'-Ende des kodierenden Genbereichs enthalten

Die Erfindung umfasst weiterhin die zu den konkret beschriebenen Nukleotidsequenzen komplementären Nukleinsäuremoleküle oder einen Abschnitt davon.

Die erfindungsgemäßen Nukleotidsequenzen ermöglichen die Erzeugung von Sonden und Primern, die zur Identifizierung und/oder Klonierung von homologen Sequenzen in anderen Zelltypen und Organismen verwendbar sind. Solche Sonden bzw. Primer umfassen gewöhnlich einen Nukleotidsequenzbereich, der unter "stringenten" Bedingungen (siehe unten) an mindestens etwa 12, vorzugsweise mindestens etwa 25, wie z.B. etwa 40, 50 oder 75 aufeinanderfolgende Nukleotide eines Sense-Stranges einer erfindungsgemäßen Nukleinsäuresequenz oder eines entsprechenden Antisense-Stranges hybridisiert.

Ein "isoliertes" Nukleinsäuremolekül wird von anderen Nukleinsäuremolekülen abgetrennt, die in der natürlichen Quelle der Nukleinsäure zugegen sind und kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Ein erfindungsgemäßes Nukleinsäuremolekül kann mittels molekularbiologischer Standard-Techniken und der erfindungsgemäß bereitgestellten Sequenzinformation isoliert werden. Beispielsweise kann cDNA aus einer geeigneten cDNA-Bank isoliert werden, indem eine der konkret offenbarten vollständigen Sequenzen oder ein Abschnitt davon als Hybridisierungssonde und Standard-Hybridisierungstechniken (wie z.B. beschrieben in Sambrook, J.; Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor, NY, 1989) verwendet werden. Überdies lässt sich ein Nukleinsäuremolekül, umfassend eine der offenbarten Sequenzen oder ein Abschnitt davon, durch Polymerasekettenreaktion isolieren, wobei die Oligonukleotidprimer, die auf der Basis dieser Sequenz erstellt wurden, verwendet werden. Die so amplifizierte Nukleinsäure kann in einen geeigneten Vektor kloniert werden und durch DNA-Sequenzanalyse charakterisiert werden. Die erfindungsgemäßen Oligonukleotide können ferner durch Standard-Syntheseverfahren, z.B. mit einem automatischen DNA-Synthesegerät, hergestellt werden.

Erfindungsgemäße Nukleinsäuresequenzen, wie SEQ ID No: 7 oder Derivate davon, Homologe oder Teile dieser Sequenzen, lassen sich beispielsweise mit üblichen Hybridisierungsverfahren oder der PCR-Technik aus anderen Bakterien, z.B. über genomische oder cDNA-Banken, isolieren. Diese DNA-Sequenzen hybridisieren unter Standardbedingungen mit den erfindungsgemäßen Sequenzen.

Unter "hybridisieren" versteht man die Fähigkeit eines Poly- oder Oligonukleotids an eine nahezu komplementäre Sequenz unter Standardbedingungen zu binden, während unter diesen Bedingungen unspezifische Bindungen zwischen nicht-komplementären Partnern unterbleiben. Dazu können die Sequenzen zu 90-100% komplementär sein. Die Eigenschaft komplementärer Sequenzen, spezifisch aneinander binden zu können, macht man sich beispielsweise in der Northern- oder Southern-Blot-Technik oder bei der Primerbindung in PCR oder RT-PCR zunutze.

Zur Hybridisierung werden vorteilhaft kurze Oligonukleotide der konservierten Bereiche verwendet. Es können aber auch längere Fragmente der erfindungsgemäßen Nukleinsäuren oder die vollständigen Sequenzen für die Hybridisierung verwendet werden. Je nach der verwendeten Nukleinsäure (Oligonukleotid, längeres Fragment oder vollständige Sequenz) oder je nachdem welche Nukleinsäureart DNA oder RNA für die Hybridisierung verwendet werden, variieren diese Standardbedingungen. So liegen beispielsweise die Schmelztemperaturen für DNA:DNA-Hybride ca 10 °C niedriger als die von DNA:RNA-Hybriden gleicher Länge.

Unter Standardbedingungen sind beispielsweise je nach Nukleinsäure Temperaturen zwischen 42 und 58°C in einer wäßrigen Pufferlösung mit einer Konzentration zwischen 0,1 bis 5 x SSC (1 X SSC = 0,15 M NaCl, 15 mM Natriumcitrat, pH 7,2) oder zusätzlich in Gegenwart von 50% Formamid wie beispielsweise 42 °C in 5 x SSC, 50% Formamid zu verstehen. Vorteilhafterweise liegen die Hybridisierungsbedingungen für DNA:DNA-Hybride bei 0,1 x SSC und Temperaturen zwischen etwa 20 °C bis 45 °C, bevorzugt zwischen etwa 30 °C bis 45 °C. Für DNA:RNA-Hybride liegen die Hybridisierungsbedingungen vorteilhaft bei 0,1 x SSC und Temperaturen zwischen etwa 30 °C bis 55 °C, bevorzugt zwischen etwa 45 °C bis 55 °C. Diese angegebenen Temperaturen für die Hybridisierung sind beispielhaft kalkulierte Schmelztemperaturwerte für eine Nukleinsäure mit einer Länge von ca. 100 Nukleotiden und einem G + C-Gehalt von 50 % in Abwesenheit von Formamid. Die experimentellen Bedingungen für die DNA-Hybridisierung sind in einschlägigen Lehrbüchern der Genetik, wie beispielsweise Sambrook et al., "Molecular Cloning", Cold Spring Harbor Laboratory, 1989, beschrieben und lassen sich nach dem Fachmann bekannten Formeln beispielsweise abhängig von der Länge der Nukleinsäuren, der Art der Hybride oder dem G + C-Gehalt berechnen. Weitere Informationen zur Hybridisierung kann der Fachmann folgenden Lehrbüchern entnehmen: Ausubel et al. (eds), 1985, Current Protocols in Molecular Biology, John Wiley & Sons, New York; Hames and Higgins (eds), 1985, Nucleic Acids Hybridization: A Practical Approach, IRL Press at Oxford University Press, Oxford; Brown (ed), 1991, Essential Molecular Biology: A Practical Approach, IRL Press at Oxford University Press, Oxford.

Die "Hybridisierung" kann insbesondere unter stringenten Bedingungen erfolgen. Solche Hybridisierungsbedingungen sind beispielsweise bei Sambrook, J., Fritsch, E.F., Maniatis, T., in: Molecular Cloning (A Laboratory Manual), 2. Auflage, Cold Spring Harbor Laboratory Press, 1989, Seiten 9.31-9.57 oder in Current Protocols in Molecular Biology, John Wiley & Sons, N.Y. (1989), 6.3.1-6.3.6 beschrieben.

Unter "stringenten" Hybridisierungs-Bedingungen werden insbesondere verstanden: Die Inkubation bei 42°C über Nacht in einer Lösung bestehend aus 50 % Formamid, 5 x SSC (750 mM NaCl, 75 mM Tri-Natrium Citrat), 50 mM Natrium Phosphat (pH7,6), 5x Denhardt Lösung, 10% Dextransulfat und 20 g/ml denaturierte, gescheerte Lachsspermien-DNA, gefolgt von einem Waschschritt der Filter mit 0,1x SSC bei 65°C.

Gegenstand der Erfindung sind auch Derivate der konkret offenbarten oder ableitbaren Nukleinsäuresequenzen.

So können weitere erfindungsgemäße Nukleinsäuresequenzen z.B. von SEQ ID NO:7 abgeleitet sein und sich davon durch Addition, Substitution, Insertion oder Deletion einzelner oder mehrerer Nukleotide unterscheiden, aber weiterhin für Polypeptide mit dem gewünschten Eigenschaftsprofil kodieren.

Erfindungsgemäß umfasst sind auch solche Nukleinsäuresequenzen, die sogenannte stumme Mutationen umfassen oder entsprechend der Codon-Nutzung eins speziellen Ursprungs- oder Wirtsorganismus, im Vergleich zu einer konkret genannten Sequenz verändert sind, ebenso wie natürlich vorkommende Varianten, wie z.B. Spleißvarianten oder Allelvarianten, davon.

Gegenstand sind ebenso durch konservative Nukleotidsubstutionen (d.h. die betreffende Aminosäure wird durch eine Aminosäure gleicher Ladung, Größe, Polarität und/oder Löslichkeit ersetzt) erhältliche Sequenzen.

Gegenstand der Erfindung sind auch die durch Sequenzpolymorphismen von den konkret offenbarten Nukleinsäuren abgeleiteten Moleküle. Diese genetischen Polyphismen können zwischen Individuen innerhalb einer Population aufgrund der natürlichen Variation existieren. Diese natürlichen Variationen bewirken üblicherweise eine Varianz von 1 bis 5 % in der Nukleotidsequenz eines Gens.

Unter Derivaten der erfindungsgemäßen Nukleinsäuresequenz mit der Sequenz SEQ ID NO: 7 sind beispielsweise Allelvarianten zu verstehen, die mindestens 60 % Homologie auf der abgeleiteten Aminosäureebene, bevorzugt mindestens 80 % Homologie, ganz besonders bevorzugt mindestens 90 % Homologie über den gesamten Sequenzbereich aufweisen (bezüglich Homologie auf Aminosäureebene sei auf obige Ausführungen zu den Polypeptiden verwiesen auf). Über Teilbereiche der Sequenzen können die Homologien vorteilhaft höher liegen.

Weiterhin sind unter Derivaten auch Homologe der erfindungsgemäßen Nukleinsäuresequenzen, insbesondere der SEQ ID NO: 7, beispielsweise pilzliche oder bakterielle Homologe, verkürzte Sequenzen, Einzelstrang-DNA oder RNA der kodierenden und nichtkodierenden DNA-Sequenz, zu verstehen. So besitzen z.B. Homologe zur der SEQ ID NO: 7 auf DNA-Ebene eine Homologie von mindestens 40 %, bevorzugt von mindestens 60 %, besonders bevorzugt von mindestens 70 %, ganz besonders bevorzugt von mindestens 80% über den gesamten in SEQ ID NO: 7 angegebenen DNA-Bereich.

Außerdem sind unter Derivaten beispielsweise Fusionen mit Promotoren zu verstehen. Die Promotoren, die den angegebenen Nukleotidsequenzen vorgeschalten sind, können durch wenigstens einen Nukleotidaustausch, wenigstens eine Insertion, Inversion und/oder Deletion verändert sein, ohne dass aber die Funktionalität bzw. Wirksamkeit der Promotoren beeinträchtigt sind. Des weiteren können die Promotoren durch Veränderung ihrer Sequenz in ihrer Wirksamkeit erhöht oder komplett durch wirksamere Promotoren auch artfremder Organismen ausgetauscht werden.

### 3.3 Erfindungsgemäße Konstrukte

Gegenstand der Erfindung sind außerdem Expressionskonstrukte, enthaltend unter der genetischen Kontrolle regulativer Nukleinsäuresequenzen eine für ein erfindungsgemäßes Polypeptid kodierende Nukleinsäuresequenz; sowie Vektoren, umfassend wenigstens eines dieser Expressionskonstrukte:

Unter einer "Expressionseinheit" wird erfindungsgemäß eine Nukleinsäure mit Expressionsaktivität verstanden, die einen Promotor, wie hierein definiert umfasst, und nach funktioneller Verknüpfung mit einer zu exprimierenden Nukleinsäure oder einem Gen, die Expression, also die Transkription und die Translation dieser Nukleinsäure oder dieses Gens reguliert. Man spricht deshalb auch in diesem Zusammenhang von einer "regulativen Nukleinsäuresequenz". Zusätzlich zum Promotor können weitere, regulative Elemente, wie z.B. Enhancer, enthalten sein.

Unter einer "Expressionskassette" oder "Expressionskonstrukt" wird erfindungsgemäß eine Expressionseinheit verstanden, die mit der zu exprimierenden Nukleinsäure oder dem zu exprimierenden Gen funktionell verknüpft ist. Im Gegensatz zu einer Expressionseinheit umfasst eine Expressionskassette somit nicht nur Nukleinsäuresequenzen, welche Transkription und Translation regulieren, sondern auch die Nukleinsäuresequenzen, welche als Folge der Transkription und Translation als Protein exprimiert werden sollen.

Die Begriffe "Expression" oder "Überexpression" beschreiben im Kontext der Erfindung die Produktion bzw. Erhöhung der intrazellulären Aktivität eines oder mehrerer Enzyme in einem Mikroorganismus, die durch die entsprechende DNA kodiert werden. Dazu kann man beispielsweise ein Gen in einen Organismus einbringen, ein vorhandenes Gen durch ein anderes Gen ersetzen, die Kopienzahl des Gens bzw. der Gene erhöhen, einen starken Promotor verwenden oder ein Gen verwenden, das für ein entsprechendes Enzym mit einer hohen Aktivität kodiert und man kann gegebenenfalls diese Maßnahmen kombinieren.

Vorzugsweise umfassen solche erfindungsgemäßen Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz.

Unter einem "Promotor", einer "Nukleinsäure mit Promotoraktivität" oder einer "Promotorsequenz" wird erfindungsgemäß eine Nukleinsäure verstanden, die in funktioneller Verknüpfung mit einer zu transkribierenden Nukleinsäure die Transkription dieser Nukleinsäure reguliert.

Unter einer "funktionellen" oder "operativen" Verknüpfung versteht man in diesem Zusammenhang beispielsweise die sequentielle Anordnung einer der Nukleinsäuren mit Promotoraktivität und einer zu transkribierenden Nukleinsäuresequenz und gegebenenfalls weiterer regulativer Elemente, wie zum Beispiel Nukleinsäuresequenzen, die die Transkription von Nukleinsäuren gewährleisten, sowie zum Beispiel einen Terminator, derart, dass jedes der regulativen Elemente seine Funktion bei der Transkription der Nukleinsäuresequenz erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen, in denen die zu transkribierende Nukleinsäuresequenz hinter (d.h. am 3'-Ende) der Promotorsequenz positioniert wird, so dass beide Sequenzen kovalent miteinander verbunden sind. Dabei kann der Abstand zwischen der Promotorsequenz und der transgen zu exprimierende Nukleinsäuresequenz geringer als 200 Basenpaare, oder kleiner als 100 Basenpaare oder kleiner als 50 Basenpaare sein.

Neben Promotoren und Terminator sind als Beispiele weiterer regulativer Elemente zu nennen Targeting-Sequenzen, Enhancer, Polyadenylierungssignale, selektierbare Marker, Amplifikationssignale, Replikationsursprünge und dergleichen. Geeignete regulatorische Sequenzen sind z.B. beschrieben in Goeddel, Gene Expression Technology: Methods in Enzymology 185, Academic Press, San Diego, CA (1990).

Erfindungsgemäße Nukleinsäurekonstrukte umfassen insbesondere Sequenz SEQ ID NO: 7 oder Derivate und Homologe davon, sowie die von SEQ ID NO: 8 ableitbaren Nukleinsäuresequenzen, die mit einem oder mehreren Regulationssignalen vorteilhafterweise zur Steuerung, z.B. Erhöhung, der Genexpression operativ oder funktionell verknüpft wurden.

Zusätzlich zu diesen Regulationssequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Das Nukleinsäurekonstrukt kann aber auch einfacher aufgebaut sein, das heißt es wurden keine zusätzlichen Regulationssignale vor die kodierende Sequenz (wie z.B. SEQ ID NO: 7 oder seine Homologen) insertiert und der natürliche Promotor mit seiner Regulation wurde nicht entfernt. Stattdessen wird die natürliche Regulationssequenz so mutiert, dass keine Regulation mehr erfolgt und die Genexpression gesteigert wird.

Ein bevorzugtes Nukleinsäurekonstrukt enthält vorteilhafterweise auch eine oder mehrere der schon erwähnten "Enhancer" Sequenzen, funktionell verknüpft mit dem Promotor, die eine erhöhte Expression der Nukleinsäuresequenz ermöglichen. Auch am 3'-Ende der DNA-Sequenzen können zusätzliche vorteilhafte Sequenzen inseriert werden, wie weitere regulatorische Elemente oder Terminatoren. Die erfindungsgemäßen Nukleinsäuren können in einer oder mehreren Kopien im Konstrukt enthalten sein. Im Konstrukt können noch weitere Marker, wie Antibiotikaresistenzen oder Auxotrophien komplementierende Gene, gegebenenfalls zur Selektion auf das Konstrukt enthalten sein.

Beispiele geeigneter Regulationssequenzen sind in Promotoren wie cos-, tac-, trp-, tet-, trp-tet-, Ipp-, lac-, Ipp-lac-, lacl^{q-,} T7-, T5-, T3-, gal-, trc-, ara-, rhaP (rhaP_{BAD})SP6-, lambda-P_{R}- oder im lambda-P_{L}-Promotor enthalten, die vorteilhafterweise in gram-negativen Bakterien Anwendung finden. Weitere vorteilhafte Regulationssequenzen sind beispielsweise in den gram-positiven Promotoren amy und SPO2, in den Hefe- oder Pilzpromotoren ADC1, MFalpha , AC, P-60, CYC1, GAPDH, TEF, rp28, ADH enthalten. Es können auch künstliche Promotoren für die Regulation verwendet werden.

Das Nukleinsäurekonstrukt wird zur Expression in einem Wirtsorganismus vorteilhafterweise in einen Vektor, wie beispielsweise einem Plasmid oder einem Phagen inseriert, der eine optimale Expression der Gene im Wirt ermöglicht. Unter Vektoren sind außer Plasmiden und Phagen auch alle anderen dem Fachmann bekannten Vektoren, also z.B. Viren, wie SV40, CMV, Baculovirus und Adenovirus, Transposons, IS-Elemente, Phasmide, Cosmide, und lineare oder zirkuläre DNA zu verstehen. Diese Vektoren können autonom im Wirtsorganismus repliziert oder chromosomal repliziert werden. Diese Vektoren stellen eine weitere Ausgestaltung der Erfindung dar.

Geeignete Plasmide sind beispielsweise in E. coli pLG338, pACYC184, pBR322, pUC18, pUC19, pKC30, pRep4, pHS1, pKK223-3, pDHE19.2, pHS2, pPLc236, pMBL24, pLG200, pUR290, pIN-III¹¹³-B1, λgt11 oder pBdCl, in Streptomyces pIJ101, pIJ364, pIJ702 oder pIJ361, in Bacillus pUB110, pC194 oder pBD214, in Corynebacterium pSA77 oder pAJ667, in Pilzen pALS1, pIL2 oder pBB116, in Hefen 2alphaM, pAG-1, YEp6, YEp13 oder pEMBLYe23 oder in Pflanzen pLGV23, pGHlac⁺, pBIN19, pAK2004 oder pDH51. Die genannten Plasmide stellen eine kleine Auswahl der möglichen Plasmide dar. Weitere Plasmide sind dem Fachmann wohl bekannt und können beispielsweise aus dem Buch Cloning Vectors (Eds. Pouwels P. H. et al. Elsevier, Amsterdam-New York-Oxford, 1985 , ISBN 0 444 904018) entnommen werden.

In einer weiteren Ausgestaltungsform des Vektors kann der das erfindungsgemäße Nukleinsäurekonstrukt oder die erfindungsgemäße Nukleinsäure enthaltende Vektor auch vorteilhafterweise in Form einer linearen DNA in die Mikroorganismen eingeführt werden und über heterologe oder homologe Rekombination in das Genom des Wirtsorganismus integriert werden. Diese lineare DNA kann aus einem linearisierten Vektor wie einem Plasmid oder nur aus dem Nukleinsäurekonstrukt oder der erfindungsgemäßen Nukleinsäure bestehen.

Für eine optimale Expression heterologer Gene in Organismen ist es vorteilhaft die Nukleinsäuresequenzen entsprechend des im Organismus verwendeten spezifischen "codon usage" zu verändern. Der "codon usage" läßt sich anhand von Computerauswertungen anderer, bekannter Gene des betreffenden Organismus leicht ermitteln.

Die Herstellung einer erfindungsgemäßen Expressionskassette erfolgt durch Fusion eines geeigneten Promotors mit einer geeigneten kodierenden Nukleotidsequenz sowie einem Terminator- oder Polyadenylierungssignal. Dazu verwendet man gängige Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley Interscience (1987) beschrieben sind.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden.

### 3.4 Erfindungsgemäß brauchbare Mikroorganismen

Je nach Zusammenhang kann unter dem Begriff "Mikroorganismus" der Ausgangsmikroorganismus (Wildtyp) oder ein genetisch veränderter, rekombinanter Mikroorganismus oder beides verstanden werden.

Mit Hilfe der erfindungsgemäßen Vektoren sind rekombinante Mikroorganismen herstellbar, welche beispielsweise mit wenigstens einem erfindungsgemäßen Vektor transformiert sind und zur Produktion der erfindungsgemäßen Polypeptide eingesetzt werden können. Vorteilhafterweise werden die oben beschriebenen erfindungsgemäßen rekombinanten Konstrukte in ein geeignetes Wirtssystem eingebracht und exprimiert. Dabei werden vorzugsweise dem Fachmann bekannte geläufige Klonierungs- und Transfektionsmethoden, wie beispielsweise Co-Präzipitation, Protoplastenfusion, Elektroporation, retrovirale Transfektion und dergleichen, verwendet, um die genannten Nukleinsäuren im jeweiligen Expressionssystem zur Expression zu bringen. Geeignete Systeme werden beispielsweise in Current Protocols in Molecular Biology, F. Ausubel et al., Hrsg., Wiley Interscience, New York 1997, oder Sambrook et al. Molecular Cloning: A Laboratory Manual. 2. Aufl., Cold Spring Harbor Laboratory, Cold Spring Harbor Laboratory Press, Cold Spring Harbor, NY, 1989 beschrieben.

Als rekombinante Wirtsorganismen für die erfindungsgemäße Nukleinsäure oder dem Nukleinsäurekonstrukt kommen prinzipiell alle prokaryontischen oder eukaryontischen Organismen in Frage. Vorteilhafterweise werden als Wirtsorganismen Mikroorganismen wie Bakterien, Pilze oder Hefen verwendet. Vorteilhaft werden gram-positive oder gram-negative Bakterien, bevorzugt Bakterien der Familien Enterobacteriaceae, Pseudomonadaceae, Rhizobiaceae, Streptomycetaceae oder Nocardiaceae, besonders bevorzugt Bakterien der Gattungen Escherichia, Pseudomonas, Streptomyces, Nocardia, Burkholderia, Salmonella, Agrobacterium oder Rhodococcus verwendet. Ganz besonders bevorzugt ist die Gattung und Art Escherichia coli. Weitere vorteilhafte Bakterien sind darüber hinaus in der Gruppe der alpha-Proteobacterien, beta-Proteobacterien oder gamma-Proteobacterien zu finden.

Der Wirtsorganismus oder die Wirtsorganismen gemäß der Erfindung enthalten dabei vorzugsweise mindestens eine der in dieser Erfindung beschriebenen Nukleinsäuresequenzen, Nukleinsäurekonstrukte oder Vektoren, die für ein Enzym mit Esteraseaktivität gemäß obiger Definition kodieren.

Die im erfindungsgemäßen Verfahren verwendeten Organismen werden je nach Wirtsorganismus in dem Fachmann bekannter Weise angezogen bzw. gezüchtet. Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, Spurenelemente wie Eisen-, Mangan-, Magnesiumsalze und gegebenenfalls Vitamine enthält, bei Temperaturen zwischen 0 °C und 100 °C, bevorzugt zwischen 10 °C bis 60 °C unter Sauerstoffbegasung angezogen. Dabei kann der pH der Nährflüssigkeit auf einen festen Wert gehalten werden, das heißt während der Anzucht reguliert werden oder nicht. Die Anzucht kann "batch"-weise, "semi batch"-weise oder kontinuierlich erfolgen. Nährstoffe können zu beginn der Fermentation vorgelegt oder semikontinuierlich oder kontinuierlich nachgefüttert werden. Das Keton kann direkt zur Anzucht gegeben werden oder vorteilhaft nach Anzucht. Die Enzyme können nach dem in den Beispielen beschriebenen Verfahren aus den Organismen isoliert werden oder als Rohextrakt für die Reaktion verwendet werden.

### 3.5 Rekombinante Herstellung der Esterase:

Gegenstand der Erfindung sind weiterhin Verfahren zur rekombinanten Herstellung erfindungsgemäße Polypeptide oder funktioneller, biologisch aktiver Fragmente davon, wobei man einen Polypeptide-produzierenden Mikroorganismus kultiviert, gegebenenfalls die Expression der Polypeptide induziert und diese aus der Kultur isoliert. Die Polypeptide können so auch in großtechnischem Maßstab produziert werden, falls dies erwünscht ist.

Die erfindungsgemäß hergestellten Mikroorganismen können kontinuierlich oder diskontinuierlich im batch- Verfahren (Satzkultivierung) oder im fed batch (Zulaufverfahren) oder repeated fed batch Verfahren (repetitives Zulaufverfahren) kultiviert werden. Eine Zusammenfassung über bekannte Kultivierungsmethoden ist im Lehrbuch von Chmiel (Bioprozeßtechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) zu finden.

Das zu verwendende Kulturmedium hat in geeigneter Weise den Ansprüchen der jeweiligen Stämme zu genügen. Beschreibungen von Kulturmedien verschiedener Mikroorganismen sind im Handbuch "Manual of Methods für General Bacteriology" der American Society für Bacteriology (Washington D. C., USA, 1981) enthalten.

Diese erfindungsgemäß einsetzbaren Medien umfassen gewöhnlich eine oder mehrere Kohlenstoffquellen, Stickstoffquellen, anorganische Salze, Vitamine und/oder Spurenelemente.

• Bevorzugte Kohlenstoffquellen sind Zucker, wie Mono-, Di- oder Polysaccharide. Sehr gute Kohlenstoffquellen sind beispielsweise Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen, wie Melassen, oder andere Nebenprodukte der Zucker-Raffinierung zu den Medien geben. Es kann auch vorteilhaft sein, Gemische verschiedener Kohlenstoffquellen zuzugeben. Andere mögliche Kohlenstoffquellen sind Öle und Fette wie z. B. Sojaöl. Sonnenblumenöl. Erdnußöl und Kokosfett, Fettsäuren wie z. B. Palmitinsäure, Stearinsäure oder Linolsäure, Alkohole wie z. B. Glycerin, Methanol oder Ethanol und organische Säuren wie z. B. Essigsäure oder Milchsäure.

Stickstoffquellen sind gewöhnlich organische oder anorganische Stickstoffverbindungen oder Materialien, die diese Verbindungen enthalten. Beispielhafte Stickstoffquellen umfassen Ammoniak-Gas oder Ammoniumsalze, wie Ammoniumsulfat, Ammoniumchlorid, Ammoniumphosphat, Ammoniumcarbonat oder Ammoniumnitrat, Nitrate, Harnstoff, Aminosäuren oder komplexe Stickstoffquellen, wie Maisquellwasser, Sojamehl, Sojaprotein, Hefeextrakt, Fleischextrakt und andere. Die Stickstoffquellen können einzeln oder als Mischung verwendet werden.

Anorganische Salzverbindungen, die in den Medien enthalten sein können, umfassen die Chlorid-, Phosphor- oder Sulfatsalze von Calcium, Magnesium, Natrium, Kobalt, Molybdän, Kalium, Mangan, Zink, Kupfer und Eisen.

Als Schwefelquelle können anorganische schwefelhaltige Verbindungen wie beispielsweise Sulfate, Sulfite, Dithionite, Tetrathionate, Thiosulfate, Sulfide aber auch organische Schwefelverbindungen, wie Mercaptane und Thiole, verwendet werden.

Als Phosphorquelle können Phosphorsäure, Kaliumdihydrogenphosphat oder Dikaliumhydrogenphosphat oder die entsprechenden Natrium haltigen Salze verwendet werden.

Chelatbildner können zum Medium gegeben werden, um die Metallionen in Lösung zu halten. Besonders geeignete Chelatbildner umfassen Dihydroxyphenole, wie Catechol oder Protocatechuat, oder organische Säuren, wie Citronensäure.

Die erfindungsgemäß eingesetzten Fermentationsmedien enthalten üblicherweise auch andere Wachstumsfaktoren, wie Vitamine oder Wachstumsförderer, zu denen beispielsweise Biotin, Riboflavin, Thiamin, Folsäure, Nikotinsäure, Panthothenat und Pyridoxin gehören. Wachstumsfaktoren und Salze stammen häufig von komplexen Medienkomponenten, wie Hefeextrakt, Melassen, Maisquellwasser und dergleichen. Dem Kulturmedium können überdies geeignete Vorstufen zugesetzt werden. Die genaue Zusammensetzung der Medienverbindungen hängt stark vom jeweiligen Experiment ab und wird für jeden spezifischen Fall individuell entschieden. Information über die Medienoptimierung ist erhältlich aus dem Lehrbuch "Applied Microbiol. Physiology, A Practical Approach" (Hrsg. P.M. Rhodes, P.F. Stanbury, IRL Press (1997) S. 53-73, ISBN 0 19 963577 3). Wachstumsmedien lassen sich auch von kommerziellen Anbietern beziehen, wie Standard 1 (Merck) oder BHI (Brain heart infusion, DIFCO) und dergleichen.

Sämtliche Medienkomponenten werden, entweder durch Hitze (20 min bei 1,5 bar und 121°C) oder durch Sterilfiltration, sterilisiert. Die Komponenten können entweder zusammen oder nötigenfalls getrennt sterilisiert werden. Sämtliche Medienkomponenten können zu Beginn der Anzucht zugegen sein oder wahlfrei kontinuierlich oder chargenweise hinzugegeben werden.

Die Temperatur der Kultur liegt normalerweise zwischen 15°C und 45°C, vorzugsweise bei 25°C bis 40°C und kann während des Experimentes konstant gehalten oder verändert werden. Der pH-Wert des Mediums sollte im Bereich von 5 bis 8,5, vorzugsweise um 7,0 liegen. Der pH-Wert für die Anzucht läßt sich während der Anzucht durch Zugabe von basische Verbindungen wie Natriumhydroxid, Kaliumhydroxid, Ammoniak bzw. Ammoniakwasser oder saure Verbindungen wie Phosphorsäure oder Schwefelsäure kontrollieren. Zur Kontrolle der Schaumentwicklung können Antischaummittel, wie z. B. Fettsäurepolyglykolester, eingesetzt werden. Zur Aufrechterhaltung der Stabilität von Plasmiden können dem Medium geeignete selektiv wirkende Stoffe, wie z. B. Antibiotika, hinzugefügt werden. Um aerobe Bedingungen aufrechtzuerhalten, werden Sauerstoff oder Sauerstoff-haltige Gasmischungen, wie z. B. Umgebungsluft, in die Kultur eingetragen. Die Temperatur der Kultur liegt normalerweise bei 20°C bis 45°C und. Die Kultur wird so lange fortgesetzt, bis sich ein Maximum des gewünschten Produktes gebildet hat. Dieses Ziel wird normalerweise innerhalb von 10 Stunden bis 160 Stunden erreicht.

Die Fermentationsbrühe wird anschließend weiterverarbeitet. Je nach Anforderung kann die Biomasse ganz oder teilweise durch Separationsmethoden, wie z. B. Zentrifugation, Filtration, Dekantieren oder einer Kombination dieser Methoden aus der Fermentationsbrühe entfernt oder vollständig in ihr belassen werden.

Die Zellen können auch, falls die Polypeptide nicht in das Kulturmedium sezerniert werden, aufgeschlossen und das Produkt nach bekannten Proteinisolierungsverfahren aus dem Lysat gewonnen. Die Zellen können wahlweise durch hochfrequenten Ultraschall, durch hohen Druck, wie z.B. in einer French-Druckzelle, durch Osmolyse, durch Einwirkung von Detergenzien, lytischen Enzymen oder organischen Lösungsmitteln, durch Homogenisatoren oder durch Kombination mehrerer der aufgeführten Verfahren aufgeschlossen werden.

Eine Aufreinigung der Polypeptide kann mit bekannten, chromatographischen Verfahren erzielt werden, wie Molekularsieb-Chromatographie (Gelfiltration), wie Q-Sepharose-Chromatographie, lonenaustausch-Chromatographie und hydrophobe Chromatographie, sowie mit anderen üblichen Verfahren wie Ultrafiltration, Kristallisation, Aussalzen, Dialyse und nativer Gelelektrophorese. Geeignete Verfahren werden beispielsweise in Cooper, F. G., Biochemische Arbeitsmethoden, Verlag Walter de Gruyter, Berlin, New York oder in Scopes, R., Protein Purification, Springer Verlag, New York, Heidelberg, Berlin beschrieben.

Vorteilhaft kann es sein, zur Isolierung des rekombinanten Proteins Vektorsysteme oder Oligonukleotide zu verwenden, die die cDNA um bestimmte Nukleotidsequenzen verlängern und damit für veränderte Polypeptide oder Fusionsproteine kodieren, die z.B. einer einfacheren Reinigung dienen. Derartige geeignete Modifikationen sind beispielsweise als Anker fungierende sogenannte "Tags", wie z.B. die als Hexa-Histidin-Anker bekannte Modifikation oder Epitope, die als Antigene von Antikörpern erkannt werden können (beschrieben zum Beispiel in Harlow, E. and Lane, D., 1988, Antibodies: A Laboratory Manual. Cold Spring Harbor (N.Y.) Press). Diese Anker können zur Anheftung der Proteine an einen festen Träger, wie z.B. einer Polymermatrix, dienen, die beispielsweise in einer Chromatographiesäule eingefüllt sein kann, oder an einer Mikrotiterplatte oder an einem sonstigen Träger verwendet werden kann.

Gleichzeitig können diese Anker auch zur Erkennung der Proteine verwendet werden. Zur Erkennung der Proteine können außerdem übliche Marker, wie Fluoreszenzfarbstoffe, Enzymmarker, die nach Reaktion mit einem Substrat ein detektierbares Reaktionsprodukt bilden, oder radioaktive Marker, allein oder in Kombination mit den Ankern zur Derivatisierung der Proteine verwendet werden.

### 3.6 Erfindungsgemäße Anwendungen der Esterasen

Gegenstand der Erfindung sind auch Verfahren zur enantioselektiven Esterhydrolyse unter Verwendung der Esterase, wobei die Esterase mit einem Stereoisomerengemisch eines optisch aktiven Esters der Formel I in Kontakt gebracht wird und die bei der stereoselektiven Hydrolyse eines der beiden Stereoisomere anfallenden optisch aktiven Verbindungen und/oder das nicht hydrolysierte Esterenantiomer aus dem Reaktionsmedium gewonnen wird. Die Esterase kann aber auch solche Ester der Formel I hydrolysieren, die nicht optisch aktiv sind.

Gegenstand der Erfindung sind auch Verfahren zur enantioselektiven Umesterung, wobei ein Stereoisomerengemisch eines optisch aktiven Alkohols der Formel II mit einem Ester der Formel I in Gegenwart der Esterase in Kontakt gebracht wird und das nicht umgesetzte Alkoholstereoisomer aus dem Reaktionsmedium gewonnen wird, oder ein Stereoisomerengemisch eines optisch aktiven Esters der Formel I mit einem Alkohol der Formel II in Gegenwart der Esterase in Kontakt gebracht wird und ein Stereoisomer des im Ester enthaltenen optisch aktiven Alkohols aus dem Reaktionsmedium gewonnen wird. Vorzugsweise werden zur Umesterung Vinylester als Acylierungsmittel für einen optisch aktiven Alkohol verwendet. Dies ist deshalb vorteilhaft, weil die Alkoholfunktion des Vinylesters nach der Umsetzung durch Tautomerisierung nicht mehr für die Rückreaktion zur Verfügung steht. Die Esterase katalysiert auch Umesterungsprozesse, bei denen weder der Ester noch der Alkohol optisch aktiv sind.

Bevorzugte Substrate zur Esterhydrolyse sind Ester des Ethanols, Propanols, Butanols und besonders bevorzugt Butinylester (Butinolester, Ester des 1-Methyl-prop-2-ynols) mit Carbonsäuren wie beispielsweise Essigsäure, Propansäure, Buttersäure, Pentansäure, Hexansäure, Heptansäure, Octansäure, Milchsäure, 2-Ethylhexansäure, 3-Methylbuttersäure, Methoxyessigsäure, 2-Methylpropionsäure, 2-Butensäure, 3-Chlorpropionsäure und 2-Methylpentansäure. Besonders bevorzugt sind Buttersäure-(Butinylbutyrat) und Methylbuttersäurebutinylester.

Bevorzugte Alkohole bei der Umesterung sind Ethanol, Propanol und Butanol, besonders bevorzugt ist Butinol.

Bevorzugte Ester bei der Umesterung sind Vinylester, wie beispielsweise Essigsäure-, Propionsäure- und Buttersäurevinylester.

Als Reaktionsmedium werden in oben stehenden Verfahren organische Lösungsmittel verwendet, wie beispielsweise Alkane, Ether, Toluol, Dioxan, Methylisobutylketon, Methyl-tertiär-butylether (MTBE) und dergleichen. Bei der Esterhydrolyse können auch Gemische aus der verwendeten Pufferlösung und organischen Lösungsmitteln wie beispielsweise MTBE und Heptan oder Toluol verwendet werden.

Die Racematspaltung, d. h. die Enantioselektivität, und Reaktionsgeschwindigkeit sind über Größe und Hydrophobizität des Säureteils beeinflussbar.

Die erfindungsgemäße Reaktion wird vorzugsweise bei Raumtemperatur bei einem pH-Wert von 6 bis 9, besonders bevorzugt bei einem pH-Wert von 7,0 bis 7,4 durchgeführt. Dabei kann die Esterase in Form von isoliertem oder aufgereinigtem Enzym, Zellen des die Esterase exprimierenden Mikroorganismus, dessen Kulturüberstand, Zelllysat oder Extrakt, oder als immobilisierte Esterase eingesetzt werden. Die Reaktionsprodukte können aus der Reaktionslösung durch dem Fachmann bekannte chemische oder physikalische Trennverfahren isoliert werden. Die Esterase kann aus dem Reaktionsgemisch durch Membranfiltration isoliert werden.

Die Immobilisierung der Esterase kann mit Hilfe von Polyacrylamid, Alginsäure oder Carragenen erfolgen. Die Esterase lässt sich auch mittels bekannter Methoden kovalent oder durch Absorption an passende Carrier binden. Vorzugsweise wird die Esterase durch Lyophilisation auf Kieselgur oder durch Ammoniumsulfatfällung immobilisiert.

### Experimenteller Teil

Werden keine anderen Angaben gemacht, so können die im Rahmen der vorliegenden Erfindung durchgeführten Klonierungsschritte, wie z.B. Restriktionsspaltungen, Agarose Gelelektrophorese, Reinigung von DNA-Fragmenten, Transfer von Nukleinsäuren auf Nitrozellulose und Nylonmembranen, Verknüpfen von DNA-Fragmenten, Transformation von Mikroorganismen, Anzucht von Mikrorganismen, Vermehrung von Phagen und Sequenzanalyse rekombinanter DNA wie bei Sambrook et al. (1989) a.a.O. beschrieben durchgeführt werden.

### Beispiel 1

### Selektion einer Esterase exprimierenden Mutante von Pseudomonas glumae

Ausgangsstamm für das Screening war der Lipase-Produzent Pseudomonas (Burkholderia) glumae LU8093. Mit der von diesem Stamm produzierten Lipase lassen sich eine ganze Reihe interessanter Reaktionen durchführen (Balkenhohl, F. et al., J. prakt. Chem. 339, (1997), 381-384). Milchsäureester, Methylbuttersäureester und Phenylessigsäureester sind jedoch kein Substrat für die Lipase und können auch sonst nicht von diesem Stamm hydrolysiert werden.

Die Hydrolyseprodukte sind jedoch als Kohlenstoffquelle verwertbar. Es wurde deshalb nach Mutanten von LU8093 gesucht, die diese Ester hydrolysieren können und mit den Hydrolyseprodukten als Kohlenstoffquelle wachsen können. Mutanten mit neuer Esterase-Aktivität sollten sich daher durch Wachstum auf diesen Estern zu erkennen geben.

Selektionsbedingungen: LU8093 wurde auf Medium für 16 h kultiviert und durch Zentrifugation geerntet. Die Zellen wurden zweimal mit Saline gewaschen. 10⁶ Zellen wurden auf Minimalmedium Platten, die 0,5 bzw. 1,0 g/l Ethylphenylacetat (EPA) als einzige Kohlenstoffquelle enthielten, ausplattiert. Zunächst zeigte sich jedoch kein Wachstum. Erst nach 4 bis 6 Tagen waren einzelne Kolonien zu erkennen. Deren Zahl nahm in den nächsten Tagen weiter zu.

Von den so erhaltenen Esterase-positiven Mutanten wurde die Mutante LU2023 ausgewählt. Überraschenderweise war die neue Esterase-Aktivität auch zur selektiven Hydrolyse kleiner organischer Moleküle geeignet. Am Beispiel von Butinolester wurde die selektive Hydrolyse gezeigt.

Hydrolyse kleiner organischer Moleküle geeignet. Am Beispiel von Butinolester wurde die selektive Hydrolyse gezeigt.

### Beispiel 2

### Fermentation von Pseudomonas glumae LU2023

Zur Gewinnung der Esterase wurde Pseudomonas glumae LU2023 im 14 I-Maßstab kultiviert und die aktive Biomasse geerntet.

Im Labor wurde Pseudomonas glumae LU2023 auf Agarplatten mit Mineralsalzmedium M12 mit 1 g/l EPA ausgestrichen und bei 28 °C für 36 bis 48 Stunden inkubiert. Die Platten konnten dann bei 4 °C vier Wochen gelagert werden.

Die Fermentation des Stammes wurde in einem 14 I-Fermenter Infors xxy durchgeführt. Für die Vorkultur wurden 250 ml Medium mit 2 bis 3 Pt-Ösen beimpft und 24 h bei 200 Upm und 28 °C inkubiert. Die Hauptkultur wurde unter folgenden Bedingungen durchgeführt:
Temperatur 28 °C
Zuluft 7 l/min
Rührung 600 Upm
Fermentationslaufzeit etwa 24 h
Eingebaute pH- und pO₂-Messung

Medium für Vorkultur und Hauptkultur
15 g/l Springer Hefeautolysat 65%
1,6 g/l Magnesiumsulfat x 7 Wasser
0,02 g/l Calciumchlorid x 2 Wasser
3,5 g/l Kaliumdihydrogenphosphat
3,5 g/l Dikaliumhydrogenphosphat
5 g/l di-Ammoniumhydrogenphosphat
6 ml Pluriol P2000 Antischaummittel

Die oben stehenden Inhaltsstoffe wurden in VE-Wasser gelöst und mit 25 % Ammoniaklösung den pH auf 6,5 eingestellt. 5 ml/l Trace Elements (Spurensalzlösung) und 2 g/l Glucose wurden separat sterilfiltriert.

Nach dem Sterilisieren und Komplettieren des Mediums wurden 0,5 g/l Ethylphenylacetat in den Fermenter gegeben. Während der Fermentation auftretender Schaum wurde durch die Zugabe von Pluriol P2000 eingedämmt. Die Fermentation wurde abgebrochen, wenn der pO₂-Wert im Fermenter wieder über 85 % anstieg. Der Fermenterinhalt wurde dann bei einer Temperatur von unter 15 °C und etwa 9000 bis 10000 g zentrifugiert und der Klarlauf verworfen. Die Zellmasse wurde bei -16 °C eingefroren.

### Beispiel 3

### Reinigung der Esterase aus Pseudomonas glumae LU2023

Zellen (100 ml, 50 g Feuchtmasse) von Pseudomonas glumae (LU2023) wurden in einer Glaskugelmühle (100 ml Glaskugeln, Durchmesser 0,5 mm) bei 4 °C und 3000 Upm aufgeschlossen. Nach Zentrifugation (30 min, 10000 Upm) und Waschen der Glaskugeln wurde mit dem Überstand (300 ml) eine Manganchloridfällung (pH 7 bis 7,5; 50 mM Endkonzentration) durchgeführt. Nach erneuter Zentrifugation wurde der pH des Überstandes auf 8,0 eingestellt und EDTA zu einer Konzentration von 50 mM zugegeben. Dieses Volumen wurde durch Chromatographie an Q-Sepharose (300 ml) gereinigt. Nach der Aufgabe der Probe wurde die Säule mit 50 mM Tris/HCl gespült. Die Wertfraktion wurde gesammelt und durch Ultrafiltration (100 kDa) konzentriert. Durch Molekularsiebchromatographie (5 cm Durchmesser, 90 cm Höhe; S-300 Material) wurde die Butinol hydrolysierende Esterase von einer unspezifischen Esterase getrennt. Die erhaltene aktive Fraktion war trübe und wurde erneut konzentriert. Die Esterase war offensichtlich membrangebunden. Die Membranfraktion wurde nun zunächst mit einer Protease verdaut (Trypsin, Gewichtsverhältnis 1:50 bis 1:100). Dabei verschwanden alle Proteine aus der Membranfraktion bis auf wenige Banden in der SDS-Polyacrylamidgelelektrophorese. Die Aktivität blieb erhalten. Diese Banden wurden durch eine native Gelelektrophorese (0,04% SDS) voneinander getrennt und ein Aktivitätstest in diesem nativen Gel identifizierte die Esterase. Diese wurde aus dem Gel eluiert und zeigte sich nun als saubere Bande in einer denaturierenden SDS-Polyacrylamidgelelektrophorese.

Das so gereinigte Protein wurde auf eine PVDF-Membran durch Blotten überführt und sequenziert oder die Peptide wurden nach Trypsinspaltung durch Reversed-Phase HPLC getrennt und sequenziert. Da der Aminoterminus des Proteins blockiert war, wurden nur tryptische Peptide erhalten. Die verschiedenen Aminosäuresequenzen wiesen schwache Homologien zu einer Muconatcycloisomerase, EC 5.5.1.1, aus Acinetobacter Iwoffii und Pseudomonas putida, sowie zur Lactonesterase aus Pseudomonas fluoreszens auf. Das Peptid mit der Sequenz AIDAIFAPEGV wies Homologie zu Pectinesterasen (EC 3.1.1.11) auf.

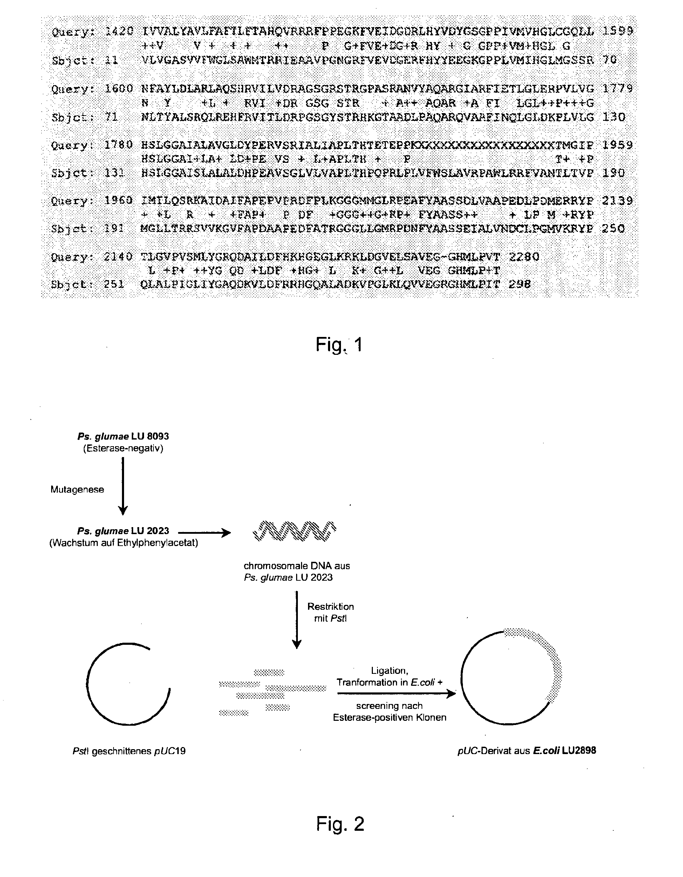

Ein Sequenz-Alignment einer erfindungsgemäßen Aminosäure-Teilsequenz mit einer Teilsequenz einer Lacton-spezifischen Esterase aus Pseudomonas fluorescens ist in Fig.1 gezeichnet.

### Beispiel 4

### Immobilisierung der Esterase

Zur Immobilisierung wurden verschiedene Methoden eingesetzt.
1. Die Esterase wurde durch Fällung mit Aceton in Gegenwart von Kieselgur weitgehend inaktiviert. 25 mg Protein wurde mit 3,5 g Kieselgur (Merck) versetzt und 1,4 l Aceton (-20 °C) wurde für 10 min zugegeben. Der beladene Träger wurde dann über eine G3 Glasnutsche abgetrennt, der Filterkuchen mit kaltem Aceton gewaschen und getrocknet.
2. Die Esterase bindet nicht an Accurel (Akzo).
3. Die Esterase konnte durch (2,3 Units/g, EPA-Test) Lyophilisation auf Kieselgur immobilisiert werden. Dazu wurde die Enzymlösung mit Kieselgur vermischt und bei -80 °C gefroren. Anschließend wurde der Feststoff durch Lyophilisation getrocknet.
4. Die Esterase wurde (454 mUnits/g, EPA-Test) durch Ammoniumsulfatfällung immobilisiert. Dazu wurde das Enzym mit einer Sättigung von 80 % an Ammoniumsulfat in Gegenwart von Kieselgur gefällt.

### Beispiel 5

### Racematspaltung mit der Esterase aus Pseudomonas glumae LU2023

### Durchführung (Standardansatz)

100 Units Esterase wurden mit 20 mMol Butinol-butyrat (Buttersäure-1-methyl-prop-2-ynyl-ester) in Phosphatpuffer (200 ml, 10 mmolar, pH 7,4) unter Rühren umgesetzt. Der pH-Wert wurde kontinuierlich gemessen und durch Zugabe von Natronlauge auf ca. pH 7,4 gehalten. Zu den in der Tabelle 1 angegebenen Zeiten wurden Proben entnommen, mit Methyl-tertiär-butylether (MTBE) zweimal ausgeschüttelt und die organische Phase durch GC (Chiraldex GTA) analysiert. Die Esterase konnte durch Membranfiltration aus dem Reaktionsgemisch entfernt werden.

Mit steigender Anreicherung des weniger bevorzugten Esterenantiomers, wurde dieses in steigendem Maße umgesetzt. Dies führte nach etwa 45 Minuten zu einem Absinken des ee-Wertes von S-Butinol im Reaktionsgemisch. Der ee-Wert des Produkts erreichte sein Maximum nach ca. 30 bis 40 min mit 84 % (83-97,9 %). Der ee-Wert des Eduktes stieg im Verlauf von 90 Minuten auf über 99 %. Der ee-Wert (Enantiomerenüberschuss) ist definiert als die Menge des bevorzugt umgesetzten Enantiomers in Prozent abzüglich der Menge des weniger bevorzugt umgesetzten Enantiomers in Prozent. Dieser Wert entspricht in den meisten Fällen der optischen Reinheit. Der pH-Abfall bis zum Zeitpunkt 30 Minuten verlief linear. Ab ca. 100 Minuten war die pH-Wert Änderung vernachlässigbar.

Die Restaktivität der Esterase in der wässrigen Phase betrug nach dem Ausschütteln noch ca. 50 %.

**Tabelle 1**

| Zeit | ee-Wert Produkt (S)-Butinol | ee-Wert Edukt (R)-Butinolester | Umsatz Ester in % (korr.) |
|---|---|---|---|
| 0 min | nd | 5,20 | nd |
| 7 min | nd | 10,20 | nd |
| 13 min | 75,50 | 20,40 | 12 |
| 20 min | 81,80 | 29,10 | 16 |
| 26 min | 83,90 | 42,00 | 22 |
| 32 min | 84,60 | 53,70 | 27 |
| 45 min | 84,00 | 78,80 | 36 |
| 70 min | 70,80 | 97,10 | 47 |
| 90 min | 69,60 | 99,10 | 52 |
| 121 min | 63,10 | 99,40 | 56 |
| 150 min | 52,00 | 99,50 | 67 |

Tabelle 1 zeigt den Enantiomerenüberschuss bei der Umsetzung von Butinol-butyrat mit der Esterase in Abhängigkeit von der Zeit. R- und S-Konfiguration definieren, nach der R/S-Nomenklatur von Cahn, Prelog und Ingold, die beiden Enantiomeren eines chiralen Moleküls. Der Umsatz ist der Anteil des umgesetzten Esters im Reaktionsgemisch.

### Beispiel 6

### Abhängigkeit der Spezifität der Esterase von der Größe und Hydrophobizität/Ladung des Säureteils des Esters

### Standardansatz

100 Units Esterase wurden mit 20 mMol Butinolester in Phosphatpuffer (200 ml, 10 mmolar, pH 7,4) unter Rühren umgesetzt. Der pH-Wert wurde kontinuierlich gemessen und durch kontinuierliche Titration auf pH 7,0 gehalten. Entnommene Proben wurden mit Methyl-tertiär-butylether (MTBE) zweimal ausgeschüttelt und die organische Phase durch GC (Chiraldex GTA) analysiert.

### Ergebnis

Die Qualität der Racematspaltung und die Reaktionsgeschwindigkeit waren von Größe und Hydrophobizität des Säureteils abhängig. Die besten Substrate für die Butinolesterase waren die Buttersäure- und Methylbuttersäure-butinolester. Lipasen sind gegenüber diesen Substraten inaktiv. Dies gilt auch für langkettige Ester wie n-Decansäurebutinylester.

**Tabelle 2**

| Säurekomponente | ee-Wert [%] | Umsatz [%] | E |
|---|---|---|---|
| Acetat | 73 (S) | 48 | 12 |
| Butyrat | 95 (S) | 36 | 67 |
| Pentanoat | 74 (S) | 47 | 13 |
| Hexanoat | 66 (S) | 44 | 8 |
| Octanoat | 64 (S) | 43 | 8 |
| 2-Ethyl-hexanoat | kein Umsatz | | |
| Phenylacetat | 51 (S) | 12 | 3 |
| 3-Phenylpropionat | 73 (S) | 44 | 11 |
| 3-Cyclohexylpropionat | 22 (S) | 18 | 2 |

Tabelle 2 zeigt den Enantiomerenüberschuss bei der Umsetzung von Estern mit der Esterase in Abhängigkeit vom Säureteil des umgesetzten Esters

### Beispiel 7

### Umesterung in organischem Medium unter Verwendung der Esterase

10 mmol rac.-Butinol und 5 mmol Buttersäurevinylester wurden in 50 ml Methyl-tertiär-butylether (MTBE) gelöst und mit 9 Units Esterase (3,3 g) geträgert auf Kieselgur versetzt und für 24 h bei RT geschüttelt. Nach Filtration wurde das Lösungsmittel entfernt und das Produktgemisch per GC charakterisiert.

Zurück blieb bei 47 % Umsatz (R)-Butinol (18 % ee) und das Butyrat des (S)-Butinols (45 % ee).

In Methylisobutylketon wurde bei 43 % Umsatz (R)-Butinol mit 16 % ee und das Butyrat des (S)-Butinols mit 52 % ee erhalten.

Tabelle 3 zeigt den Enantiomerenüberschuss bei der Umsetzung von Estern mit der Esterase in Abhängigkeit vom Säureteil des umgesetzten Esters.

**Tabelle 3**

| Ansatz Nr. | Edukt | pH-Wert | Temp. [°C] | Puffersystem Lsg. [mmol/l] | Zusatzstoffe | ee-Werte ¹⁾ |
|---|---|---|---|---|---|---|
| 8 | n-Decansäurebutinylester | 7,0 | RT | Phosphat 10 | keine | 54,37 |
| 14 | n-Pentansäurebutinylester | 7,0 | RT | Phosphat 10 | keine | 80,40 |
| 15 | 2-Ethylhexansäurebutinylester | 7,0 | RT | Phosphat 10 | keine | 81,77 |
| 16 | Butinylbutyrat | 7,0 | RT | Phosphat 10 | keine | 83,90 |
| 17 | Butinylbutyrat | 7,0 | RT | Phosphat 10 | 0,5% Triton | 80,83 |
| 18 | n-Hexansäurebutinylester | 7,0 | RT | Phosphat 10 | 0,5% Triton | 78,63 |
| 19 | n-Octansäurebutinylester | 7,0 | RT | Phosphat 10 | 0,5% Triton | 74,70 |
| 20 | Butinylbutyrat | 7,0 | RT | Phosphat 10 | 10% n-Propanol | 87,47 |
| 21 | Butinylbutyrat | 7,0 | RT | Phosphat 10 | 1 M NaCl | 85,70 |
| 23 | n-Pentansaurebutinylester | 7,0 | RT | Phosphat 10 | 0,5% Triton | 84,40 |
| 24 | Butinylbutyrat | 6,0 | RT | Phosphat 10 | keine | 85,37 |
| 25 | Butinylbutyrat | 8,0 | RT | Tris 10 | keine | 85,33 |
| 26 | Butinylbutyrat | 7,0 | 10 | Phosphat 10 | keine | 85,90 |
| 27 | Butinylbutyrat | 7,0 | 37 | Phosphat 10 | keine | 75,67 |
| 28 | 3-Methylbuttersäurebutinylester | 7,0 | RT | Phosphat 10 | keine | 90,50 |
| 29 | Methoxyessigsäurebutinylester | 7,0 | RT | Phosphat 10 | keine | 76,33 |
| 31 | Butinylbutyrat | 7,0 | RT | Phosphat 10 | keine | 85,00 |
| 32 | Butinylbutyrat | 7,0 | RT | Phosphat 10 | 2-Phasensvstem | 84,93 |
| 33 | 3-Methylbuttersäurebutinylester | 7,0 | RT | Phosphat 10 | 2-Phasensystem | 92,70 |
| 34 | 2-Methylpropionsäurebutinylester | 7,0 | RT | Phosphat 10 | keine | 89,17 |
| 35 | 2-Butensäurebutinylester | 7,0 | RT | Phosphat 10 | keine | 76,03 |
| 36 | 3-Chlorpropionsäurebutinylester | 7,0 | RT | Phosphat 10 | keine | 71,13 |
| 40 | 2-Methylpentansäurebutinylester | 7,0 | RT | Phosphat 10 | keine | 85,93 |

| | | | | | | |
|---|---|---|---|---|---|---|
| ¹⁾ Mittelwert der drei besten ee-Werte von S-Butinol | | | | | | |

### Beispiel 8:

### Genbankherstellung von Stamm LU2023 und Klonierung von LU2898

### a) Gewinnung der chromosomalen DNA:

LU2023 (vgl. Beispiele 1 und 2 oben) wurde in 100ml FP-Medium (Becton Dickinson GmbH) bei 28°C und 180Upm für 24h angezogen. Die Zellen wurden mittels Zentrifugation (2000 Upm / 5min) geerntet, in 5ml Lysepuffer 1 (0,41 M Saccharose, 0,01 M MgSO₄*7H₂O, 50 ml/L M12-Medium (10x conc.), 10 ml/L 10% KH₂PO₄ pH 6,7, 2,5 mg/ml Lysozym [kurz vor Gebrauch zugeben]) aufgenommen und ca. 4h bei 37°C inkubiert. Nach Zentrifugation (2000Upm / 20min) werden die so entstandenen Protoplasten mit 5ml Lysepuffer 1 ohne Lysozym gewaschen (2000Upm / 20min) und in 10mM TRIS-HCl pH 8.0 aufgenommen.

Nach einem weiteren Waschschritt mit 10mM TRIS-HCl pH 8.0 (3000Upm / 5min) wurde das Pellet in 4ml TE-Puffer (10mM TRIS-HCl, 1 mM EDTA, pH8) resuspendiert und mit je 0,5ml SDS (10% w/v) bzw. NaCl (5M) vermischt. Es folgte die Zugabe von 100µl Proteinase K-Lösung (200µg/ml) und einer 16-stündigen Inkubation bei 37°C. Danach wurde die Lösung TE-Puffer auf 10ml aufgefüllt. Diese Lösung wurde 1:1 mit Phenol vermischt. Nach Zentrifugation (4000Upm / 5min) wurde die obere Phase abgenommen und mit einer Mischung aus Phenol, Chloroform und iso-Amylalkohol (12:12:1) vermengt. Die obere Phase wurde abgenommen und mit einem Volumenteil (VT) Chloroform iso-Amylalkohol (24:1) vermengt. Dieser Vorgang wurde so oft wiederholt bis die obere Phase klar ist.

Aus der Oberphase wurde die DNA durch Zugabe von 2 VT Ethanol und 1/50 VT CH₃COONa (3M) bei -20°C gefällt (Dauer: ca. 30min) und durch Zentrifugation (12000Upm, 30min, 4°C) sedimentiert und in TE-Puffer aufgenommen. RNase (1ml einer 20 µg/ ml Lösung) hydrolysiert RNA innerhalb von 1h bei 37°C. Danach wird die Lösung dreimal für 1 Stunde gegen TE-Puffer bei 4°C dialysiert. In 0.4ml Aliquots wird die DNA durch Zugabe Ethanol (2 VT plus 1/3 VT LiCl, 2M) und 30-minütige Inkubation bei -20°C ausgefällt. Durch Zentrifugation (15000Upm, 30min, 4°C) sedimentierte die DNA, die anschließend getrocknet wurde. Die DNA aus einem 0.4ml Aliquot wurde in 0.5ml TE-Puffer aufgenommen.

### b) Restriktion der DNA und Klonierung

1µg der chromosomalen DNA wurden mit 120U *Pst*I bei 37°C für 180min verdaut. 0,4µg *p*UC19 (z. B. Fa. New England Biolabs) wurden mit 160U *Pst*I restringiert (1 h bei 37°C) und anschließend mit alkalischer Phosphatase dephosphoryliert (1h bei 37°C plus 15-minütige Inaktivierung bei 65°C). Mit dem Rapid-Liagtion Kit (Fa. Roche) wurden die DNA-Fragmente ligiert (2h bei Raumtemperatur). Die Ligationsansätze wurden nach Angaben des Herstellers in die transformationskompetente *Escherichia coli* sure (Fa. Stratagene) transformiert. Die Zellen wurden auf IPTG/X-Gal FP-Platten (FP-Medium mit 100µg/ml Ampicillin) ausgestrichen und für 16 Stunden bei 37°C inkubiert. Weiße Kolonien werden auf frische FP-Platten mit Ampicillin ausgestrichen.

### c) Genbankscreening

Die Kolonien wurden mit einem sterilen Samtkissen auf Whatman-Filterpapier übertragen. Die Filter wurden in 2.5ml Assaylösung (10mM TRIS*HCl, pH7.5, 0,01 % bromocresolpurple, 0,1% racemischer Ester [*rac*-Milchsäuremethylester (MEE) oder rac-Methylbuttersäureethylester oder rac-Ethylphenylacetat]) gelegt. Kolonien, deren Zellen Esteraseaktivität aufweisen, verfärben sich innerhalb von 5 Minuten von blau nach gelb. Unter den 2900 getesteten Kolonien fand sich eine, die einen starken Farbumschlag verursachte. Dieser Stamm wurde als LU2898 bezeichnet. Figur 2 zeigt eine schematische Darstellung des Klonierungsschemas von LU2898.

### Beispiel 9

### Sequenzanalyse der rekombinanten DNA von LU2898

Das Plasmid aus LU2898 besitzt eine 7.7kB große Insertion. Das Plasmid mit dem Butinolesterasegen *E.coli* LU2898 wurde durchsequenziert. Eine erste Suche in DNA-Datenbanken ergab ein offenes Leseraster (Position 1394 bis 2923) mit einer Homologie des Inserts zu hydrolytischen Enzymen.

Mit der DNA-Sequenz der Butinolesterase aus LU2898 wurden die ERGO-Datenbank (Integrated Genomics, Inc. ©1999-2004, Chicago, USA) durchsucht. Dabei wurde gefunden, dass nur der Bereich von 1384 bis 2397 bp des Butinolesterasegens zu bekannten Hydrolasen homolog ist.

Bei dem Bereich von 1384 bis 2397 bp handelt es sich offensichtlich um die DNA der gesuchten Butinolesterase. Ein weiterer guter Hinweis ist das für Serin-Carboxylesterasen typische GXSXG-Motiv, welches sich bei der Butinolesterase aus LU2023 an den Aminosäurepositionen 127-131 befindet.

Etwa ab Position 2400 des Inserts bricht die gute Übereinstimmung zwischen dem Butinolesterasegen aus LU2898 und der Hydrolase aus *B. cepacia* ab. Im weiteren Leseraster findet sich eine sehr deutliche Homologie zu den 3'-Enden von NADP- Reduktasen.

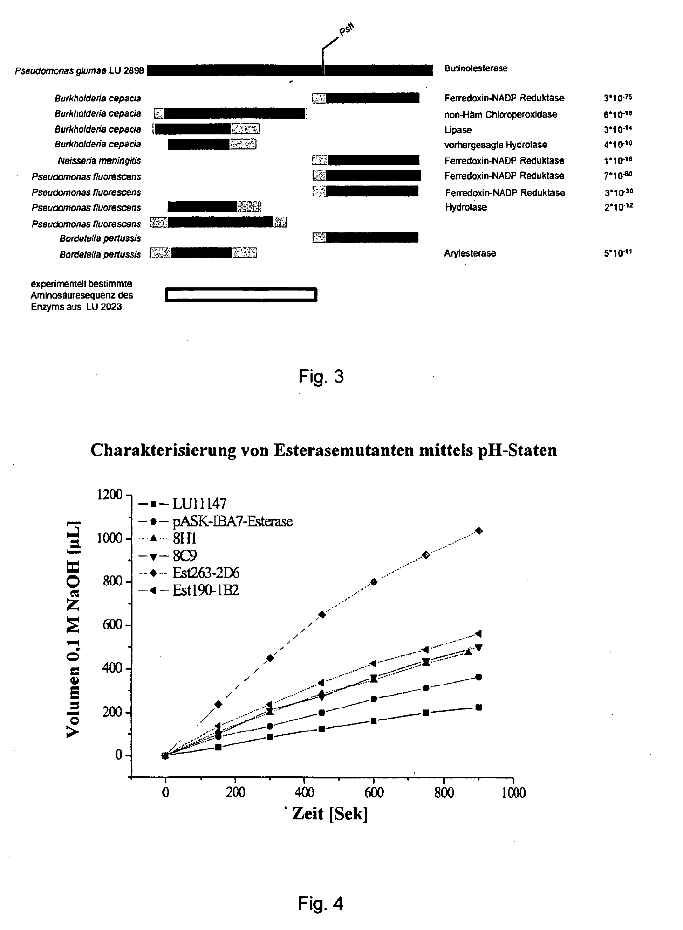

Diese Ergebnisse werden durch beiliegende Figur 3 veranschaulicht. Figur 3 fasst die ERGO-Analysen zusammen. Der abrupte Übergang der Homologien in der Butinolesterase aus LU2898 geht einher mit einer *Pst*I-Schnittstelle. Schnittstellen für das Restriktionsenzym *Pst*I wurden bei der gewählten Klonierungsstrategie nicht erwartet. Mit diesem Enzym wurde die chromosomale DNA aus LU2023 für die Herstellung der Plasmidbank vollständig geschnitten. Nach einer vollständigen Hydrolyse sollten keine internen Erkennungsstellen für *Pst*I mehr vorhanden sein. Der Befund der *Pst*I Schnittstellen im Plasmid von LU2898 lässt sich damit erklären, dass unterschiedliche Genfragmente, die durch die *Pst*I-Restriktion entstanden sind, während der Plasmidkonstruktion miteinander verbunden wurden, und diese schließlich in die *Pst*I-Schnittstelle von pUC19 ligiert wurden. Dies bedeutet, dass das Leseraster, das der Butinolesterase zugeordnet wurde, möglicherweise nur einen Teil des tatsächlichen Gens aus Ps. glumae LU2023 darstellt. An der Pstl-site ist das Butinolesterasegen unterbrochen und anschließend mit einem Fragment aus einem NADP-Reduktase-Gen verbunden worden.

Diese Hypothese wird durch Daten der Proteinsequenzierung unterstützt. Die Butinolesterase aus *Ps*. *glumae* LU2023 ist gereinigt worden. Durch Edman-Abbau wurde die Aminosäuresequenz des Proteins bestimmt. Bis auf wenige N- und C-terminale Aminosäuren ist die komplette Sequenz bekannt. Sie stimmt mit der aus den DNA-Daten von LU2898 abgeleiteten Aminosäuresequenz aus dem vorderen Teil der Butinolesterase überein. Das experimentell ermittelte Molekulargewicht der Butinolesterase von 41.3 kDa ist deutlich geringer als der Wert, der sich aus der DNA-Sequenz des rekombinanten Butinolesterasegens aus LU2898 ableitet. Nach den Daten der DNA-Sequenz müsste die Butinolesterase ein Molekulargewicht von 55 kDa haben.

Mit Hilfe der Datenbank-Analyse wurden Hinweise darauf gefunden, dass das Butinolesterase nicht in seiner ganzen Länge kloniert wurde. Das Plasmid aus LU2898 enthält nicht das 3'-Ende des Butinolesterasegens.

### Beispiel 10

### Auffinden des Klons LU11147 enthaltend einer verkürzte 335AS Butinolesterase-Mutante .

Die Sequenzdaten des Plasmids aus LU2898 wurden genutzt um folgende PCR-Primer zu entwerfen.

Mit diesen Oligonukleotiden wurde mittels PCR die DNA aus LU2898 amplifiziert, die die oben erläuterte Homologie zu Esterase besitzt.

PCR-Bedingungen:

| | |
|---|---|
| 5 µL | 10*Taq-Polymerase-Puffer |
| 125 ng | Primer Breu0811 |
| 125 ng | Primer Breu0812 |
| 2 µL | dNTP (10 mM) |
| 50 ng | Plasmid-DNA aus LU11147 |
| 2.5 µl | DMSO |
| ad 50 µL | H₂O |
| hot start mit: 0.5 U | Taq-DNA-Polymerase (Fa. Roche Diagnostics) |

### Temperaturprogramm PCR:

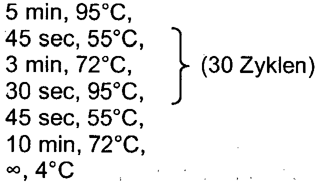

Nach einer Restriktion mit *Hind*III und *Xba*I wird das PCR-Produkt in einen entsprechend vorbereiteten *p*UC19 (z.B. Fa. New England Biolabs) ligiert.
Mit dem so hergestellten Plasmidvektor wurde anschließend *E.coli* XL1 blue transformiert. Die Ligationsansätze wurden nach Angaben des Herstellers in die transformationskompetente Escherichia coli sure (Fa. Stratagene) transformiert. Die Zellen wurden auf IPTG/X-Gal FP-Platten (FP-Medium mit 100µg/ml Ampicillin) ausgestrichen und für 16 Stunden bei 37°C inkubiert. Aus den Transformanten wurde Plasmid-DNA präpariert (Birnboim et al., Nucleic Acids Research, **1979,** 7, 1513) und mittels Kontrollrestriktion mit *Hind*III und *Xba*I auf erfolgreiche Insertion des PCR-Produktes überprüft.

### Beispiel 11

### Expression der verkürzten 335 AS Butinol Esterase

Das Esterasegen wurde anschließend in den Vektor pDHE1650 (beschrieben in WO-A-2004/050877 unter Verwendung der Schnittstellen NdeI und HindIII kloniert.

Hierzu wurde zunächst das Esterasegen unter Verwendung folgender Vorwärts- bzw. Rückwärtsprimer amplifiziert:
Breu1499 TATACATATGATCGTCCAACTGATCGCCATCGTG,
Breu1500 ATTTAAGCTTTTACTGCAGCGCGCCGGCCTGCGTGACCTC
gefolgt von einem Restriktionsverdau unter Verwendung von Ndel und HindIII. Das Insert wurde in den Vektor pDHE1650 ligiert, der zuvor mit den gleichen Restriktionsenzymen (Ndel und HindIII) vorverdaut worden war. Auf diese Weise wurde das in pDHE1650 vorliegende Platzhaltergen durch das erfindungsgemäße Esterasegen ersetzt. Zwei Transformanten wurden isoliert und die Plasmide wurden in an sich bekannter Weise durch DNA-Präzipitation extrahiert. Diese wurden mit pDHE1650-Est2 und pDHE1615-Est4 bezeichnet.

Zur Proteinexpression wurde das Plasmid, welches das Esterasegen trägt, wurde unter Verwendung der TSS-Methode ([Chung CT, Niemela SL und Miller RH, 1989. One-step preparation of competent Escherichia coli: Transformation and Storage of Bacterial Cells in the Same Solution. Proc. Natl. Acad. Sci. U.S.A. 86: 2172-2175.) in ein *Escherichia coli TG1* (DSMZ Nr. 6056) transformiert. Eine einzelne Kolonie wurde isoliert und in 5 ml LB/Amp/Tet/Spec/Cm-Medium (= LB-Medium nach Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor, NY, 1989 plus 100µg/mL Ampicillin, 10µg/mL Tetrazyklin, 100µg/mL Spectinomycin und 20µg/mL Chloramphenicol) über Nacht bei 37 °C und 250 Upm vermehrt. 100 ml LB/Amp/Tet/Spec/Cm-Medium, supplementiert mit 10mL/L Spurenelementlösung, wurde mit 500 µl der Übernacht-Kultur inokuliert und bei 37 °C und 200 Upm vermehrt. (Die Spurenelementlösung ist wie folgt zusammengesetzt: FeSO₄ * H₂O 200 mg/L, ZnSO₄ * 7H₂O 10 mg/L, MnCl₂ * 4H₂O 3 mg/L, H₃BO₃ 30 mg/L, CoCl₂ * 6H₂O 20 mg/L, CuCl₂ * 6H₂O 1 mg/L, NiCl₂ * 6H2O 2 mg/L, Na₂MoO₄ * 2H₂O 3 mg/L, Titriplex III 500 mg/L. Die Proteinexpression wurde mit 1 mM L-Rhamnose induziert, sobald die OD₆₀₀ einen Wert von 0,5 bis 0,6 erreicht hatte. Die Temperatur wurde auf 30 °C verringert, während die Rotationsgeschwindigkeit bei 200 Upm gehalten wurde. Die Kultur wurde über Nacht kultiviert. Die Zellen wurden anschließend abzentrifugiert (15 min, 4 °C, 3220 g) und das Zellpellet wurde über Nacht bei -20 °C aufbewahrt.

Für den Zellaufschluss wurde das Zellpellet (aus 50 ml TB-Kultur) in Natriumphosphatpuffer (25 mM, 154 mM Ionenstärke, pH 7,5) resuspendiert. Die Zellen wurden durch Homogenisieren (1500 bar, 2 Passagen) aufgeschlossen. Zellfragmente wurden abzentrifugiert (30 min, 4 °C, 20817 g). Der Überstand wurde auf einen Protein 200 Plus-Labchip aufgetragen und unter Verwendung des Agilent 2100 Bioanalyzers auf Protein analysiert. Die abzentrifugierten Zellfragmente wurden in 8 M Harnstoff resuspendiert, gefolgt von einer Zentrifugation (30 min, 4 °C, 20817 g), um nicht-gelöste Partikel zu entfernen. Der klare Überstand (Membranfraktion) wurde auf einen Protein 200 Plus-Labchip aufgetragen und unter Verwendung des Agilent 2100 Bioanalyzers auf Protein analysiert.

### Beispiel 12:

### Herstellung von Mutanten der Butinol Esterase (335AS)

### a) Materialien

Sämtliche verwendete Chemikalien waren für analytische Zwecke geeignet oder in einer höheren Qualitätsstufe und wurden von Sigma-Aldrich Chemie, Taufkirchen, Deutschland, Applichem (Darmstadt, Deutschland) und Carl Roth (Karlsruhe, Deutschland) bezogen.

Ein Thermocycler (Mastercycler gradient; Eppendorf, Hamburg, Deutschland) sowie Dünnwand-PCR-Röhrchen (Multi-Ultra Röhrchen; 0,2 ml; Carl Roth) wurden in sämtlichen PCR-Experimenten verwendet. Das PCR-Volumen betrug jeweils 50 µl; größere Volumina wurden in mehrfachen 50 µl PCR-Ansätzen hergestellt. Die Menge an verwendeter DNA wurde unter Verwendung eines NanoDrop Photometers (NanoDrop Technologies, Wilmington, DE) quantifiziert.

### b) Klonierung des Bunitolesterase-Gens in pASK-IBA7-Vektor:

Der pASK-IBA7-Vektor (IBA GmbH, Göttingen) (SEQ ID NO:19; Plasmid zur Proteinexpression mit N-terminalem Strep-tag) wurde unter Verwendung der folgenden Primer amplifiziert:
Vorwärtsprimer
   5'-TTTTTGCCCTCGTTATCTAGATTT-3' (SEQ ID NO:9)
Rückwärtsprimer
   5'-CCGGAATTCCGGTATCTAACTAAGCTTGACCTG-3' (SEQ ID NO:10)

Der 50 µl-PCR-Ansatz (95 °C 3 min; 1 Zyklus // 95 °C 30 sec, 56,2 °C 45 sec, 72 °C 7 Min; 30 Zyklen // 72 °C 10 Min; 1 Zyklus) enthielt: 20 pmol Vorwärtsprimer, 20 pmol Rückwärtsprimer, 0,2 mM von jedem dNTP (Roche Diagnostics GmbH, Mannheim, Deutschland), 150 ng Plasmid pASK-IBA7 und 2,5 U Pfu DNA Polymerase (Fermentas GmbH, St. Leon-Rot). Das Produkt wurde mittels Gel gereinigt und zwar unter Verwendung des QIAquick Gel Extraction Kit (Qiagen, Hilden) und unter den oben erwähnten identischen Bedingungen erneut amplifiziert. Nach Gelreinigung (QIAquick Gel Extraction Kit; Qiagen) wurden 2 µg des DNA-Produktes mit 40 U EcoRI (New England Biolabs GmbH, Frankfurt) in einem 100 µl-Reaktionsgemisch 4 Stunden bei 37 °C verdaut. Das verdaute Produkt wurde unter Verwendung des QIAquick PCR Purification Kit (Qiagen) PCR-gereinigt.

Das Butinolesterase-Gen wurde unter Verwendung folgender Primer amplifiziert:
Vorwärtsprimer
   5'-[Phos]GACCATGATTACGCCAAGCTTGC-3' (SEQ ID NO:11)
Rückwärtsprimer
   5'-CCGGAATTCCGGTCACTGCAGCGCGCCGGCCTG-3' (SEQ ID NO:12).

Der 50 µl PCR-Ansatz (95 °C 2 min; 1 Zyklus // 95 °C 30 sec, 59 °C 45 sec, 72 °C 3 min; 30 Zyklen // 72 °C 10 min; 1 Zyklus) enthielt: 20 pmol Vorwärtsprimer, 20 pmol Rückwärtsprimer, 0,2 mM von jedem dNTP (Roche Diagnostics GmbH); 150 ng Plasmid LU11147 (pUC19 Vektor, der das Butinolesterase-Gen trägt), 0,02 % DMSO und 2,5 U Pfu DNA Polymerase (Fermentas). Nach Gelreinigung (QIAquick Gel Extraction Kit; Qiagen) wurden 2 µg der produzierten DNA mit 40 U EcoRI (New England Biolabs GmbH) in einem 100 µl-Reaktionsgemisch 4 Stunden bei 37 °C verdaut. Das Verdauungsprodukt wurde unter Verwendung des QIAquick PCR Purification Kit (Qiagen) PCR-gereinigt.

Das Butinolesterase-Gen wurde in den Vektor unter Verwendung von stumpfen und EcoRI-Schnittstellen ligiert. Das Reaktionsgemisch enthielt in 20 µl: 20 ng Vektor, 120 ng Insert und 2 U T4 DNA-Ligase (Roche Diagnostics GmbH). Das Reaktionsgemisch wurde zunächst 1 Stunde bei Raumtemperatur inkubiert, gefolgt von einer Übernacht-Inkubation im Kühlschrank. Das resultierende Plasmid wurde als *pASK-IBA7-Esterase* bezeichnet.

### c) Herstellung der ersten Butinolesterase-Mutanten-Bibliothek:

Das Butinolesterase-Gen wurde zunächst mittels fehleranfälliger PCR (95 °C 2 min; 1 Zyklus // 95 °C 30 sec, 59 °C 45 sec, 72 °C 3 min; 40 Zyklen // 72 °C 10 min) amplifiziert. Folgende Primer wurden verwendet:
Vorwärtsprimer:
   5'-CGACAAAAATCTAGATAACGAGGGCAA-3' (SEQ ID NO:13)
Rückwärtsprimer:
   5'-TTCACTTCACAGGTCAAGCTTAGTTAG-3' (SEQ ID NO:14),

Das Reaktionsgemisch enthielt in 50 µl: 20 pmol Vorwärtsprimer, 20 pmol Rückwärtsprimer, 0,2 mM von jedem dNTP (Roche Diagnostics GmbH), 100 ng Plasmid pASK-IBA7-Esterase, 0,02 % DMSO, 0,1 mM MnCl₂ und 2,5 U Taq DNA Polymerase (Qiagen). Nach Gelreinigung (QIAquick Gel Extraction Kit; Qiagen) wurden 4 µg DNA mit 40 U EcoRI (New England Biolabs GmbH) und 30 U Xbal (New England Biolabs GmbH) doppelt verdaut. Das Produkt wurde PCR-gereinigt (QIAquick PCR-Purification Kit; Qiagen) bevor es in das mit EcoRI und Xbal vorverdaute Plasmid pASK-IBA7 ligiert wurde. Die Mutantenbibliothek wurde in *E*. *coli* XL2Blue (Stratagene, Amsterdam) unter Verwendung der TSS-Methode [Chung CT, Niemela SL und Miller RH,1989. One-step preparation of competent Escherichia coli: Transformation and Storage of Bacterial Cells in the Same Solution. Proc. Natl. Acad. Sci. U.S.A. 86: 2172-2175.] transformiert.

### d) Expression der Butinolesterase-Mutanten-Bibliothek:

Kolonien auf LBₐₘₚ-Platten (= LB-Medium nach Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor, NY, 1989 plus 10g/L Agar und 100µg/mL Ampicillin) wurden unter Verwendung von Zahnstochern gepickt und in Mikrotiterplatten mit 96 Vertiefungen, enthaltend 150 µl LSₐₘₚ-Medium überfüht. Nach Kultivierung in einem Mikroplattenschüttler (Multitron II; Infors GmbH, Einsbach; 37 °C, 900 Upm und 70 % Feuchtigkeit) wurden -5 µl einer jeden Kultur mit System Duetz (Kühner, Birsfelden, Schweiz) in Platten mit 2ml-Vertiefungen, enthaltend 550 µl TB-Medium mit 100µg/mL Ampicillin (Becton Dickinson GmbH) übertragen. Die Klone wurden in einem Multitron II-Schüttler (Infors GmbH; 30 °C und 500 Upm und 70% Feuchtigkeit) 8 Stunden kultiviert. Die Proteinexpression wurde durch Zugabe von 50 µl TB enthaltend 2,4 µg/ml Anhydrotetracyclin (IBA GmbH) in jede Vertiefung induziert. Die Klone wurden weitere 6 Stunden im Multitron II-Schüttler (Infors GmbH; 30 °C und 500 Upm) 8 Stunden kultiviert.

### e) pH-Indikator-Assay (96-well Format):

Eine 50 µl-TB-Kultur aus jeder Vertiefung wurde auf Testplatten mit 96 Vertiefungen unter Verwendung eines automatischen 96-Kanal-Pipettierers Multimek 96 (Beckman Coulter, Krefeld) übertragen. Die hydrolytische Reaktion wurde durch Zugabe von 100 µl Substratlösung (25 mM NaH₂PO₄-Puffer, pH 7,5, 154 mM Ionenstärke, eingestellt mit NaCl, enthaltend 0,05 % Triton X-100, 7,5 µg/ml Flurorescein-Natrium und 285 mM But-3-yn-2-yl butyrat) in jede Vertiefung der Testplatte unter Verwendung von Multimek 96 (Beckman Coulter) gestartet. Die Substratlösung wurde vor der Verwendung 5 min kräftig geschüttelt. Die Kinetik der Abnahme des Fluoreszenzsignals (Extinktion 485 nm, Emission 520 nm) wurde unter Verwendung von Tecan Safire (Tecan GmbH, Crailsheim) 10 min beobachtet. Die Aktivität wurde aus der Anfangssteigung ermittelt. Nach dem Screening von 930 Klonen wurde Klon 8H1 als verbesserte Mutante identifiziert.

### f) Zweite Butinolesterase-Mutanten-Bibliothek:

Eine zweite Butinolesterase-Mutanten-Bibliothek wurde unter exakt den gleichen Bedingungen wie oben beschrieben erzeugt, mit Ausnahme davon, dass 8H1 als parentales Gen verwendet wurde. Die Bibliothek wurde wie oben beschrieben exprimiert und gescreent. Nach einem Screening von 1116 Klonen wurde *Klon 8C9* als verbesserte Mutante identifiziert.

### g) Sättigungsmutagenese in den Aminosäurepositionen 190 und 263:

Eine Sättigungsmutagenese der Aminosäurepositionen 190 und 263 wurde unter Verwendung des folgenden modifizierten QuikChange Protokolls (Stratagene) durchgeführt. Für Aminosäureposition 190 wurden die folgenden Mutagenese-Primer verwendet:
5'-GGCATCCCGATCATGNNNCTGCAAAGCCGCAAG-3' (SEQ 10 NO:15)
5'-CTTGCGGCTTTGCAGNNNCATGATCGGGATGCC-3' (SEQ 10 NO:16)

Für die Aminosäureposition 263 wurden die folgenden Mutagenese-Primer verwendet:
5'-GGGCGCCAGGACGCGNNNCTCGATTTCCACAAG-3' (SEQ ID NO:17)
5'-CTTGTGGAAATCGAGNNNCGCGTCCTGGCGCCC-3' (SEQ ID NO:18)

Der 50 µl PCR-Ansatz (95 °C 30 sec; 1 Zyklus // 95 °C 30 sec, 55 °C 1 min, 68 °C 4 min 45 sec; 18 Zyklen) enthielt ein Gemisch aus 20 pmol von jedem Primer, 0,2 mM von jedem dNTP (Roche Diagnostics GmbH), 50 ng Plasmid 8C9 (Ausgangs-DNA) und 2,5 U Pfu Turbo DNA Polymerase (Stratagene). Nach der PCR wurde methylierte und hemi-methylierte parentale DNA mit 40 U Dpnl (New England Biolabs GmbH) 2 bis 3 Stunden bei 37 °C verdaut. Die Produkte wurden PCR-gereinigt (QIAquick PCR-Purification Kit; Qiagen), bevor sie in E. coli XL2Blue (Stratagene) unter Verwendung der TSS-Methode [Chung CT, a.a.O.] transformiert wurden.

Die zwei Bibliotheken wurden in identischer Weise wie oben beschrieben exprimiert und gescreent. Nach einem Screening on 186 Klonen für jede Bibliothek wurden zwei Klone als verbesserte Mutanten identifiziert. Diese wurden als *Est190-1B2* und *Est263-*2D6 bezeichnet.

### h) Proteinexpression in Schüttelkolben:

Übernacht-Kulturen wurden in 5 ml LBₐₘₚ-Medium unter Verwendung eines Multitron-II-Schüttlers (Infors GmbH; 37 °C, 250 Upm) durch Picken einer frisch transformierten Kolonie gezüchtet. 100 ml TBₐₘₚ-Medium wurde mit 800 µl der Übernacht-Kultur inokuliert und in einem Multitron-II-Schüttler (Infors GmbH; 37 °C, 200 Upm) kultiviert. Nachdem die OD₆₀₀ Werte von 0,5 bis 0,6 erreicht hatte, wurde die Proteinexpression durch Zugabe von 25 µl Anhydrotetracyclin (Stammlösung 0,2 mg/ml, in DMF) induziert. Die Kultur wurde weitere 12 Stunden gezüchtet (Multitron-II-Schüttler, Infors GmbH; 30 °C, 200 Upm). Die Zellen wurden bei 3220 g, 4 °C, 30 min abzentrifugiert und die Feuchtzellmasse wurde bestimmt. Die Zellpellets wurden bis zur weiteren Charakterisierung bei -20 °C aufbewahrt.

### i) Proteincharakterisierung unter Verwendung von pH-stat:

Die hydrolytische Aktivität der Butinolesterase wurde unter Verwendung von pH-stat (716 DMS Titrino, Deutsche Metrohm GmbH & Co., Filderstadt) gemessen.

Die Zellpellets wurden zunächst in einem geeigneten Volumen von Natriumphosphatpuffer (25mM Natriumdihydrogenphosphatpuffer, pH 7,5, 154 mM Ionenstärke, eingestellt mit NaCl) resuspendiert, um eine Zellkonzentration von 0,1 g Feuchtzellmasse/ml zu erhalten. 20 ml Substratlösung, enthaltend Natriumphosphatpuffer (25 mM NaH₂PO₄-Puffer, pH 7,5, 154 mM Ionenstärke, eingestellt mit NaCl), 0,05 % Triton X-100 und 350 mM But-3-yn-2-yl-butyrat wurden hergestellt. Die Substratlösung wurde vor der Verwendung 5 min kräftig geschüttelt.

Vor dem Start der Hydrolysereaktion wurde der pH der Substratlösung (20 ml) mit 1 N NaOH auf pH 7,5 eingestellt. Die Reaktion wurde durch Zugabe von 0,01 g Feuchtzellmasse gestartet. Der pH wurde bei pH 7,5 während der Biokonversion durch Titration mit 0,1 N NaOH gehalten. Die hydrolytische Aktivität wurde mit der Titrationsrate von NaOH (Volumen, zugesetzt pro Zeiteinheit) korreliert.

Die Ergebnisse sind in beiliegender Figur 4 dargestellt. Die in folgender Tabelle aufgelisteten Enzyme bzw. Mutanten wurden getestet. Der Tabelle sind ebenfalls Angaben zur den jeweiligen Mutation in der Aminosäure- und Nukleinsäuresequenz zu entnehmen.

| | **Anminosäureposition** | | |
|---|---|---|---|
| **Klone** | **16** | **190** | **263** |
| pASK-IBA7-Esterase | ctg (L) | att (I) | atc (I) |
| 8H1 | ccg (P) | act (T) | atc (I) |
| 8C9 | ccg (P) | act (T) | gtc (V) |
| Est190-1B2 | ccg (P) | cgc (R) | gtc (V) |
| Est 263-2D6 | ccg (P) | act (T) | gtt (V) |

## Patentansprüche

1. Protein mit Esterase-Aktivität, umfassend eine Aminosäuresequenz gemäß SEQ ID NO: 8 oder eine davon abgeleitete Aminosäuresequenz mit einer Identität von mindestens 80% zur Ausgangssequenz, jeweils **gekennzeichnet durch** eine Polypeptidkette mit einem berechneten Molekulargewicht von etwa 38 bis 34 kDa und eine Esterase-Aktivität, die **durch** Spaltung des Esters But-3-yn-2-yl-butyrat charakterisierbar ist.

2. Protein nach Anspruch 1, umfassend eine Polypeptidkette mit einer Gesamtlänge von weniger als 350 bis 335 Aminosäuren.

3. Protein nach Anspruch 2, umfassend eine Aminosäuresequenz gemäß SEQ ID NO:8.

4. Esterase-Mutante mit Esterase-Aktivität welche wenigstens eine Mutation in einem der Aminosäuresequenzbereiche 12-20, 185-195 und 258 bis 268 von SEQ ID NO:2 oder 8 aufweist.

5. Mutante gemäß Anspruch 4, umfassend wenigstens eine Mutation in einer der Aminosäuresequenzpositionen 16, 190 und 263 von SEQ ID NO:2 oder 8.

6. Mutante gemäß Anspruch 5, umfassend wenigstens eine der folgenden Aminosäuresubstitutionen: Leu16Pro, Ile190Thr, Ile190Arg und Ile 263Val.

7. Mutante gemäß einem der Ansprüche 4 bis 6, umfassend eine Polypeptidkette mit einem berechneten Molekulargewicht von etwa 60 kDa oder weniger.

8. Protein oder Mutante gemäß einem der vorhergehenden Ansprüche, **dadurch gekennzeichnet, dass** es ausgehend von Pseudomonas glumae (Burkholderia plantarii) Lu 2023 mit der Hinterlegungsnummer DSM 13176 erhältlich ist.

9. Protein oder Mutante gemäß einem der vorhergehenden Ansprüche, **dadurch gekennzeichnet, dass** es wenigstens eine der folgenden Reaktionen katalysiert:
a) enantioselektive Hydrolyse optisch aktiver Ester der Formel I
R¹-COO-R² (I),
worin
R¹ für geradkettiges oder verzweigtes, gegebenenfalls ein- oder mehrfach substituiertes C₁-C₁₀-Alkyl, C₂-C₁₀-Alkenyl, C₂-C₁₀-Alkinyl und R² für geradkettiges oder verzweigtes, gegebenenfalls ein- oder mehrfach substituiertes C₁-C₁₀-Alkyl, C₂-C₁₀-Alkenyl, C₂-C₁₀-Alkinyl, C₇-C₁₅-Aralkyl oder für einen ein- oder mehrkernigen, gegebenenfalls ein- oder mehrfach substituierten aromatischen Rest steht,
R¹ und/oder R² wenigstens ein asymmetrisches Kohlenstoffatom umfasst; und
b) enantioselektive Umesterung eines Esters der Formel I mit einem optisch aktiven Alkohol der Formel 11
R²-OH (II),
worin R² eine der oben stehenden Bedeutungen besitzt, und gegebenenfalls wenigstens ein asymmetrisches Kohlenstoffatom aufweist.

10. Polynukleotid, das für ein Protein oder eine Mutante gemäß einem der vorhergehenden Ansprüche kodiert, sowie dessen komplementäre Polynukleotide, und die damit hybridisierbaren Nukleinsäuresequenzen.

11. Polynukleotid nach Anspruch 10, wobei das Codon in dem Bereich entsprechend der Aminosäureposition 263 von SEQ ID NO:8 ausgewählt ist unter GTT und GTC.

12. Expressionskassette, umfassend wenigstens ein Polynukleotid gemäß einem der Ansprüche 10 und 11 operativ verknüpft mit wenigstens einer regulatorischen Nukleinsäuresequenz.

13. Rekombinanter Vektor zur Transformation eines eukaryontischen oder prokaryontischen Wirts, umfassend ein Polynukleotid gemäß einem der Ansprüche 10 oder 11, oder eine Expressionskassette gemäß Anspruch 12.

14. Verfahren zur Herstellung eines Proteins oder einer Mutante gemäß einem der Ansprüche 1 bis 9, wobei man einen Mikroorganismus, welcher das Protein endogen produziert, oder einen mit einem Vektor gemäß Anspruch 13 transformierten Mikroorganismus kultiviert und das Protein aus der Kultur isoliert.

15. Verfahren gemäß Anspruch 14, wobei der Mikroorganismus Pseudomonas glumae (Burkholderia plantarii) Lu 2023 mit der Hinterlegungsnummer DSM 13176 ist.

16. Protein, erhältlich nach einem Verfahren gemäß einem der Ansprüche 14 und 15.

17. Mikroorganismus, **dadurch gekennzeichnet, dass** er einen Vektor gemäß Anspruch 13 trägt.

18. Verfahren zur enantioselektiven Esterhydrolyse unter Verwendung eines Proteins oder einer Mutante gemäß einem der Ansprüche 1 bis 9, wobei
a) das Protein oder die Mutante mit einem Stereoisomerengemisch eines optisch aktiven Esters der Formel I in Kontakt gebracht wird; und
b) die bei der stereoselektiven Hydrolyse eines der Stereoisomeren anfallenden optisch aktiven Verbindungen und/oder das nicht hydrolysierte Esterenantiomer aus dem Reaktionsmedium gewonnen wird.

19. Verfahren zur enantioselektiven Umesterung, wobei
a) ein Stereoisomerengemisch eines optisch aktiven Alkohols der Formel II mit einem Ester der Formel I in Gegenwart eines Proteins oder einer Mutante gemäß einem der Ansprüche 1 bis 9 in Kontakt gebracht wird und das nicht umgesetzte Alkoholstereoisomer aus dem Reaktionsmedium gewonnen wird; oder
b) ein Stereoisomerengemisch eines optisch aktiven Esters der Formel I mit einem Alkohol der Formel II in Gegenwart eines Proteins oder einer Mutante gemäß einem der Ansprüche 1 bis 9 in Kontakt gebracht wird und ein Stereoisomer des im Ester enthaltenen optisch aktiven Alkohols aus dem Reaktionsmedium gewonnen wird.

20. Verfahren nach Anspruch 19, wobei man zur Umesterung einen Vinylester als Acylierungsmittel für einen optisch aktiven Alkohol verwendet.

21. Verfahren gemäß einem der Ansprüche 18 bis 20, wobei als Reaktionsmedium ein organisches Lösungsmittel verwendet wird.

## Claims

1. A protein having esterase activity, comprising an amino acid sequence according to SEQ ID NO: 8 or an amino acid sequence derived therefrom which is at least 80% identical to the starting sequence, **characterized in** each case by a polypeptide chain having a calculated molecular weight of about 38 to 34 kDa and an esterase activity which can be **characterized by** way of cleavage of the ester but-3-yn-2-yl butyrate.

2. The protein according to claim 1, comprising a polypeptide chain of less than 350 to 335 amino acids in total length.

3. The protein according to claim 2, comprising an amino acid sequence according to SEQ ID NO: 8.

4. An esterase mutant having esterase activity which has at least one mutation in any of the amino acid sequence regions 12-20, 185-195 and 258 to 268 of SEQ ID NO: 2 or 8.

5. The mutant according to claim 4, comprising at least one mutation in any of the amino acid sequence positions 16, 190 and 263 of SEQ ID NO: 2 or 8.

6. The mutant according to claim 5, comprising at least one of the following amino acid substitutions: Leu16Pro, Ile190Thr, Ile190Arg and Ile263Val.

7. The mutant according to any of claims 4 to 6, comprising a polypeptide chain having a calculated molecular weight of about 60 kDa or less.

8. The protein or mutant according to any of the preceding claims, wherein said protein or mutant is obtainable starting from Pseudomonas glumae (Burkholderia plantarii) Lu 2023 with deposition number DSM 13176.

9. The protein or mutant according to any of the preceding claims, wherein said protein or mutant catalyzes at least one of the following reactions:
a) enantioselective hydrolysis of optically active esters of the formula I
R¹- COO-R² (I),
in which
R¹ is straight-chain or branched, optionally mono- or polysubstituted C₁-C₁₀₋alkyl, C₂-C₁₀-alkenyl, C₂-C₁₀-alkynyl, and R² is straight-chain or branched, optionally mono- or polysubstituted C₁-C₁₀-alkyl, C₂-C₁₀-alkenyl, C₂-C₁₀-alkynyl, C₇-C₁₅-aralkyl or a mono- or polynuclear, optionally mono- or polysubstituted aromatic radical,
R¹ and/or R² comprise (s) at least one asymmetric carbon atom; and
b) enantioselective transesterification of an ester of the formula I with an optically active alcohol of the formula II
R²-OH (II),
in which R² has one of the above meanings and optionally has at least one asymmetric carbon atom.

10. A polynucleotide encoding a protein or a mutant according to any of the preceding claims, and also polynucleotides complementary thereto and the nucleic acid sequences hybridizable therewith.

11. The polynucleotide according to claim 10, wherein the codon in the region corresponding to amino acid position 263 of SEQ ID NO: 8 is selected from among GTT and GTC.

12. An expression cassette comprising at least one polynucleotide according to either of claims 10 and 11 operatively linked to at least one regulatory nucleic acid sequence.

13. A recombinant vector for transforming a eukaryotic or prokaryotic host, comprising a polynucleotide according to either of claims 10 and 11 or an expression cassette according to claim 12.

14. A process for preparing a protein or a mutant according to any of claims 1 to 9, which comprises culturing a microorganism which produces said protein endogenously or a microorganism transformed with a vector according to claim 13 and isolating said protein from the culture.

15. The process according to claim 14, in which the microorganism is Pseudomonas glumae (Burkholderia plantarii) Lu 2023 with deposition number DSM 13176.

16. A protein obtainable by a process according to either of claims 14 and 15.

17. A microorganism, carrying a vector according to claim 13.

18. A process for enantioselective ester hydrolysis using a protein or a mutant according to any of claims 1 to 9, which process comprises
a) contacting said protein or said mutant with a stereoisomer mixture of an optically active ester of the formula I; and
b) obtaining the optically active compounds produced by stereoselective hydrolysis of any of said stereoisomers and/or the nonhydrolyzed ester enantiomer from the reaction medium.

19. A process for enantioselective transesterification, which comprises
a) contacting a stereoisomer mixture of an optically active alcohol of the formula II with an ester of the formula I in the presence of a protein or a mutant according to any of claims 1 to 9 and obtaining the unreacted alcohol stereoisomer from the reaction medium; or
b) contacting a stereoisomer mixture of an optically active ester of the formula I with an alcohol of the formula II in the presence of a protein or a mutant according to any of claims 1 to 9 and obtaining a stereoisomer of the optically active alcohol comprised in said ester from the reaction medium.

20. The process according to claim 19, which comprises using for transesterification a vinyl ester as acylating agent for an optically active alcohol.

21. The process according to any of claims 18 to 20, which comprises using an organic solvent as reaction medium.

## Revendications

1. Protéine ayant une activité d'estérase, comprenant une séquence d'acides aminés selon SEQ ID NO:8 ou une séquence d'acides aminés qui en dérive, ayant une identité d'au moins 80 % avec la séquence de départ, chacune étant **caractérisée par** une chaîne polypeptidique ayant une masse moléculaire calculée d'environ 38 à 34 kDa et une activité d'estérase caractérisable par clivage de l'ester butyrate de but-3-yn-2-yle.

2. Protéine selon la revendication 1, comprenant une chaîne polypeptidique ayant une longueur totale inférieure à 350 à 335 acides aminés.

3. Protéine selon la revendication 2, comprenant une séquence d'acides aminés selon SEQ ID NO:8.

4. Mutant d'estérase ayant une activité d'estérase, qui comprend au moins une mutation dans l'un des domaines de séquence d'acides aminés 12-20, 185-195 et 258 à 268 de SEQ ID NO:2 ou 8.

5. Mutant selon la revendication 4, comprenant au moins une mutation sur l'une des positions de séquence d'acides aminés 16, 190 et 263 de SEQ ID NO:2 ou 8.

6. Mutant selon la revendication 5, comprenant au moins l'une des substitutions suivantes d'acides aminés : Leu16Pro, Ile190Thr, Ile190Arg et Ile263Val.

7. Mutant selon l'une des revendications 4 à 6, comprenant une chaîne polypeptidique ayant une masse moléculaire calculée d'environ 60 kDa ou moins.

8. Protéine ou mutant selon l'une des revendications précédentes, **caractérisé en ce qu'**il peut être obtenu à partir de Pseudomonas glumae (Burkholderia plantarii) Lu2023, ayant le numéro de dépôt DSM 13176.

9. Protéine ou mutant selon l'une des revendications précédentes, **caractérisé en ce qu'**il catalyse au moins l'une des réactions suivantes :
a) hydrolyse énantio-sélective d'esters optiquement actifs de formule I
R¹ -COO-R² (I)
dans laquelle
R¹ représente un groupe alkyle en C₁-C₁₀, alcényle en C₂-C₁₀, alcynyle en C₂-C₁₀ à chaîne droite ou ramifiée, éventuellement mono- ou polysubstitué, et R² représente un groupe alkyle en C₁-C₁₀, alcényle en C₂-C₁₀, alcynyle en C₂-C₁₀, aralkyle en C₇-C₁₅, à chaîne droite ou ramifiée, éventuellement mono- ou polysubstitué, ou un radical aromatique mono- ou polycyclique, éventuellement mono- ou polysubstitué,
R¹ et R² comprennent au moins un atome de carbone asymétrique, et
b) transestérification énantio-sélective d'un ester de formule I avec un alcool optiquement actif de formule II
R²-OH (II)
dans laquelle R² a l'une des significations données ci-dessus, et comprend éventuellement au moins un atome de carbone asymétrique.

10. Polynucléotide qui code pour une protéine ou un mutant selon l'une des revendications précédentes, ainsi que les polynucléotides qui en sont complémentaires, et les séquences d'acides nucléiques pouvant s'y hybrider.

11. Polynucléotide selon la revendication 10, dans lequel le codon, dans le domaine correspondant à la position d'acide aminé 263 de SEQ ID NO:8, est choisi parmi GTT et GTC.

12. Cassette d'expression comprenant au moins un polynucléotide selon l'une des revendications 10 et 11 fonctionnellement lié à au moins une séquence d'acide nucléique régulatrice.

13. Vecteur recombinant pour la transformation d'un hôte eucaryote ou procaryote, comprenant un polynucléotide selon l'une des revendications 10 ou 11 ou une cassette d'expression selon la revendication 12.

14. Procédé de fabrication d'une protéine ou d'un mutant selon l'une des revendications 1 à 9, dans lequel on cultive un microorganisme qui produit la protéine d'une manière endogène, ou un microorganisme transformé avec un vecteur selon la revendication 13, et on isole la protéine de la culture.

15. Procédé selon la revendication 14, dans lequel le microorganisme est Pseudomonas glumae (Burkholderia plantarii) Lu2023, ayant le numéro de dépôt DSM 13176.

16. Protéine pouvant être obtenue par un procédé selon l'une des revendications 14 et 15.

17. Microorganisme, **caractérisé en ce qu'**il porte un vecteur selon la revendication 13.

18. Procédé d'hydrolyse énantio-sélective d'esters par utilisation d'une protéine ou d'un mutant selon l'une des revendications 1 à 9, dans lequel
a) on met la protéine ou le mutant en contact avec un mélange de stéréo-isomères d'un ester optiquement actif de formule I et,
b) on récupère du milieu réactionnel les composés optiquement actifs obtenus lors de l'hydrolyse stéréo-sélective de l'un des stéréo-isomères, et/ou l'énantiomère d'ester non hydrolysé.

19. Procédé de transestérification énantio-sélective, dans lequel
a) on met en contact un mélange de stéro-isomères d'un alcool optiquement actif de formule II avec un ester de formule I en présence d'une protéine ou d'un mutant selon l'une des revendications 1 à 9, et on récupère du milieu réactionnel le stéréo-isomère d'alcool n'ayant pas réagi, ou
b) on met en contact un mélange de stéréo-isomères d'un ester optiquement actif de formule I avec un alcool de formule II en présence d'une protéine ou d'un mutant selon l'une des revendications 1 à 9, et on récupère du milieu réactionnel un stéréo-isomère de l'alcool optiquement actif contenu dans l'ester.

20. Procédé selon la revendication 19, dans lequel on utilise pour la transestérification un ester vinylique en tant qu'agent d'acylation pour un alcool optiquement actif.

21. Procédé selon l'une des revendications 18 à 20, dans lequel on utilise en tant que milieu réactionnel un solvant organique.
